# EUROPEAN PATENT SPECIFICATION

(11) **EP 2 066 626 B1**
(45) Date of publication and mention of the grant of the patent: **17.03.2010**
(21) Application number: 07804896.4
(22) Date of filing: 04.09.2007
(51) Int. Cl.: C07D 205/04, A61K 31/397

(54) **AZETIDINE DERIVATIVES AS MUSCARINIC RECEPTOR ANTAGONISTS**
AZETIDINDERIVATE ALS ANTAGONISTEN DES MUSKARINREZEPTORS
DERIVES D'AZETIDINE UTILISES EN TANT QU'ANTAGONISTES DU RECEPTEUR MUSCARINIQUE

(30) Priority: 22.09.2006 US 826629 P
(43) Date of publication of application: 10.06.2009
(73) Proprietor: Pfizer Limited, Sandwich, Kent CT13 9NJ (GB)
(72) Inventor: GLOSSOP, Paul, Alan, Sandwich, Kent CT13 9NJ (GB); STRANG, Ross, Sinclair, Sandwich, Kent CT13 9NJ (GB); WATSON, Christine, Anne, Louise, Sandwich, Kent CT13 9NJ (GB); WOOD, Anthony, Sandwich, Kent CT13 9NJ (GB)
(74) Representative: Laurent, Claire
(86) International application number: PCT/IB2007/002593
(87) International publication number: WO 2008/035157

(56) References cited:
- EP-A- 0 948 964
- WO-A-2007/034325
- JP-A- 11 100 366

## Description

This invention relates to compounds of general formula (I): 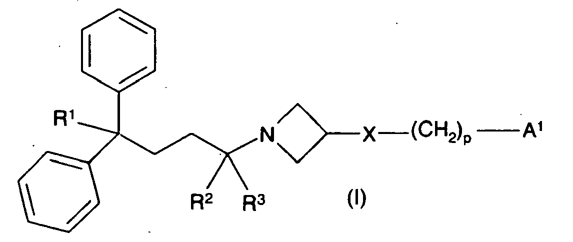 in which R¹, R², R³, A¹, X and p have the meanings indicated below, and to processes and intermediates for the preparation of, compositions containing and the uses of such derivatives.

Cholinergic muscarinic receptors are members of the G-protein coupled receptor super-family and are further divided into 5 subtypes, M₁ to M₅. Muscarinic receptor sub-types are widely and differentially expressed in the body. Genes have been cloned for all 5 sub-types and of these, M₁, M₂ and M₃ receptors have been extensively pharmacologically characterized in animal and human tissue. M₁ receptors are expressed in the brain (cortex and hippocampus), glands and in the ganglia of sympathetic and parasympathetic nerves. M₂ receptors are expressed in the heart, hindbrain, smooth muscle and in the synapses of the autonomic nervous system. M₃ receptors are expressed in the brain, glands and smooth muscle. In the airways, stimulation of M₃ receptors evokes contraction of airway smooth muscle leading to bronchoconstriction, while in the salivary gland M₃ receptor stimulation increases fluid and mucus secretion leading to increased salivation. M₂ receptors expressed on smooth muscle are understood to be pro-contractile while pre-synaptic M₂ receptors modulate acetylcholine release from parasympathetic nerves. Stimulation of M₂ receptors expressed in the heart produces bradycardia.
Short and long-acting muscarinic antagonists are used in the management of asthma and COPD; these include the short acting agents Atrovent® (ipratropium bromide) and Oxivent® (oxitropium bromide) and the long acting agent Spiriva® (tiotropium bromide). These compounds produce bronchodilation following inhaled administration. In addition to improvements in spirometric values, anti-muscarinic use in chronic obstructive pulmonary disease (COPD) is associated with improvements in health status and quality of life scores.
As a consequence of the wide distribution of muscarinic receptors in the body, significant systemic exposure to muscarinic antagonists is associated with effects such as dry mouth, constipation, mydriasis, urinary retention (all predominantly mediated via blockade of M₃ receptors) and tachycardia (mediated by blockade of M₂ receptors). A commonly reported side-effect following inhaled administration of therapeutic dose of the current, clinically used non-selective muscarinic antagonists is dry-mouth and while this is reported as only mild in intensity it does limit the dose of inhaled agent given.

Accordingly, there is still a need for improved M₃ receptor antagonists that would have an appropriate pharmacological profile, for example in term of potency, pharmacokinetics or duration of action. In this context, the present invention relates to novel M₃ receptor antagonists. In particular, there is a need for M₃ receptor antagonists that would have a pharmacological profile suitable for an administration by the inhalation route.

The scientific literature discloses many compounds having a muscarinic receptor antagonist activity.
EP0948964A1 discloses compounds of formula 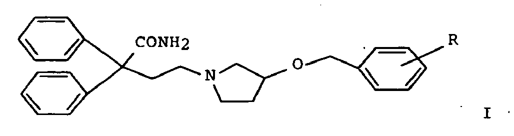 in which R denotes a hydrogen atom, a halogen atom or a lower alkoxy group.

The invention relates to a compound of formula (I) 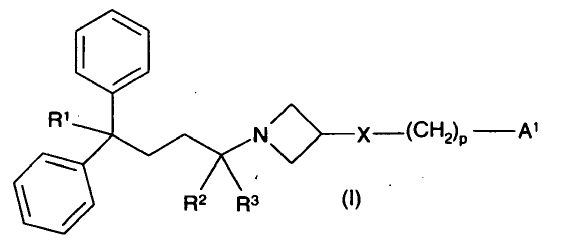 wherein,
- R¹ is CN or CONH₂;
- R² and R³ are methyl, or, R² and R³ may also together form with the carbon atom to which they are linked a cyclopentane ring;
- X is NH or S;
- p is 0 or 1;
- A¹ is selected from
   a) phenyl optionally substituted with 1, 2 or 3 groups independently selected from halo, CN, CF₃, OR⁴_{,} SR⁴, OCF₃, (C₁-C₄)alkyl and phenyl optionally substituted with OH;
   b) naphthyl optionally substituted with 1 or 2 groups independently selected from halo, CN, CF₃, OR⁴, SR⁴, OCF₃ and (C₁-C₄)alkyl;
   c) a 9 or 10-membered bicyclic aromatic heterocyclic group, containing from 1 to 3 heteroatoms independently selected from or S or N, said heterocyclic group being optionally substituted with 1 or 2 substituents selected from OR⁴, (C₁-C₄)alkyl and halo;
- R⁴ is H or (C₁-C₄)alkyl;
or the pharmaceutically acceptable salts or solvates thereof.

In the here above general formula (I), (C₁-C₄)alkyl denote a straight-chain or branched group containing 1, 2, 3 or, 4 carbon atoms. This also applies if they carry substituents or occur as substituents of other radicals, for example in O-(C₁-C₄)alkyl radicals, S-(C₁-C₄)alkyl radicals etc.... Examples of suitable (C₁-C₄)alkyl radicals are methyl, ethyl, *n*-propyl, *iso*-propyl, *n*-butyl, *iso*-butyl, *sec*-butyl, *tert*-butyl.... Examples of suitable O-(C₁-C₄)alkyl radicals are methoxy, ethoxy, *n*-propyloxy, *iso*-propyloxy, *n*-butyioxy, *iso*-butyloxy, *sec*-butyloxy and *tert*-butyloxy....

Examples of 9 or 10-membered bicyclic aromatic heterocyclic group, containing from 1 to 3 heteroatoms independently selected from O, S or N are indolyl, isoindolyl, quinolyl, isoquinolyl, benzofuranyl, isobenzofuranyl, benzothienyl, isobenzothienyl, quinazolyl, quinoxalyl, phthalazinyl, benzothiazolyl, benzoxazolyl, benzisothiazolyl, benzisoxazolyl, benzimidazolyl, indazolyl, benzotriazolyl, benzoxadiazolyl, benzisoxadiazolyl, benzothiadiazolyl and benzisothiadiazolyl.

Preferred 9 or 10-membered bicyclic aromatic heterocyclic groups are benzoxazolyl, benzothiazolyl, benzofuranyl, benzothienyl, isoquinolyl and quinolyl. Benzoxazolyl is particularly preferred.

Halo denotes a halogen atom selected from the group consisting of fluoro, chloro, bromo and iodo. Preferred halo groups are fluoro or chloro.

In the above compounds of formula (I) and in the intermediates useful for their preparation, the following definitions are preferred:
Preferably, R¹ is CONH₂.
Preferably, R⁴ is H or CH_{3:}
Preferably, A¹ is phenyl optionally substituted with 1 to 3 groups, independently selected from F, Cl, CF₃, OH, OCH₃, OCF₃ and CH₃. More preferably, A¹ is phenyl optionally substituted with 1 to 2 groups independently selected from F, Cl, CF₃, OH, OCH₃, OCF₃ and CH₃.
Even more preferably, A¹ is phenyl optionally substituted with 1 to 2 groups independently selected from F, Cl and OH.
Preferably; R² and R³ are methyl.
In a preferred embodiment, p is 0 and X is S.
In another preferred embodiment, p is 1 and X is NH.

Preferred compounds according to the invention are:
5-(3-Benrylamino-azetidin-1-yl)-5-methyl-2,2-diphenyl-hexanenitrile;
5-(3-Benzylamino-azetidin-1-yl)-5-methyl-2,2-diphenyl-hexanoic acid amide;
5-[3-(2-Chloro-3-hydroxy-benzylamino)-azetidin-1-yl]-5-methyl-2,2-diphenyl-hexanoic acid amide;
5-[3-(5-Chloro-2-hydroxy-benzylamino)-azetidin-1-yl]-5-methyl-2,2-diphenyl-hexanoic acid amide;
5-[3-(5-Fluoro-2-hydroxy-benzylamino)-azetidin-1-yl]-5-methyl-2,2-diphenyl-hexanoic acid amide;
5-[3-(3-Hydroxy-benzylamino)-azetidin-1-yl]-5-methyl-2,2-diphenyl-hexanoic acid amide;
5-[3-(5-Fluoro-2-hydroxy-benzylamino)-azetidin-1-yl]-5-methyl-2,2-diphenyl-hexanenitrile;
5-[3-(5-Chloro-2-hydroxy-benrylamino)-azetidin-1-yl]-5-methyl-2,2-diphenyl-hexanenitrile;
5-[3-(2-Hydroxy-benzylamino)-azetidin-1-yl]-5-methyl-2,2-diphenyl-hexanoic acid amide;
5-[3-(4-Fluoro-3-hydroxy-benzylamino)-azetidin-1-yl]-5-methyl-2,2-diphenyl-hexanoic acid amide;
5-[3-(4-Chloro-3-methoxy-benzylamino)-azetidin-1-yl]-5-methyl-2,2-diphenyl-hexanoic acid amide;
5-[3-(4-Chloro-3-hydroxy-benzylamino)-azetidin-1 -yi]-5-methyl-2,2-diphenyl-hexanoic acid amide;
5-[3-(3-Methoxy-phenylsulfanyl)-azetidin-1-yl]-5-methyl-2,2-diphenyl-hexanenitrile;
5-[3-(3-Methoxy-phenylsulfanyl)-azetidin-1-yl]-5-methyl-2,2-diphenyl-hexanoic acid amide, 5-[3-(3-Hydroxy-phenylsulfanyl)-azetidin-1-yl]-5-methyl-2,2-diphenyl-hexanoic acid amide,
5-[3-(3-Chloro-4-hydroxy-benzylamino)-azetidin-1-yl]-5-methyl-2,2-diphenyl-hexanoic acid amide, and,
5-[3-(4-hydroxy-benzylamino)-azetidin-1-yl]-5-methyl-2,2-diphenyl-hexanoic acid amide.
or the pharmaceutically acceptable salts or solvates thereof.
The invention also relates to processes for the preparation of the compounds of formula (I) as well as intermediates useful for their preparation. In particular, the invention relates to the intermediates (VIII), (IX) and (X): 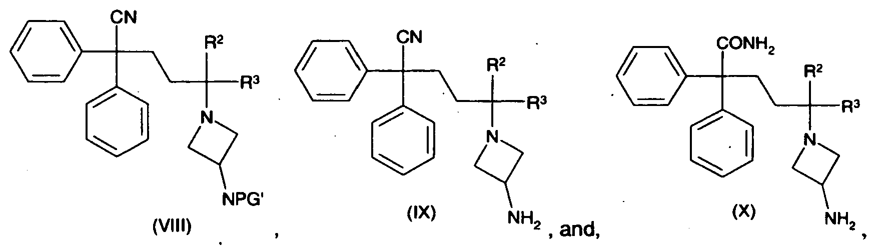 wherein R² and R³ are as defined for compounds of formula (I) and PG' is a suitable amine protecting group such as phthalimide or benzyl and is preferably phthalimide.

Compounds of formula (I) may be prepared in a variety of ways. The routes below illustrate one such way of preparing these compounds; the skilled person will appreciate that other routes may be equally as practicable. 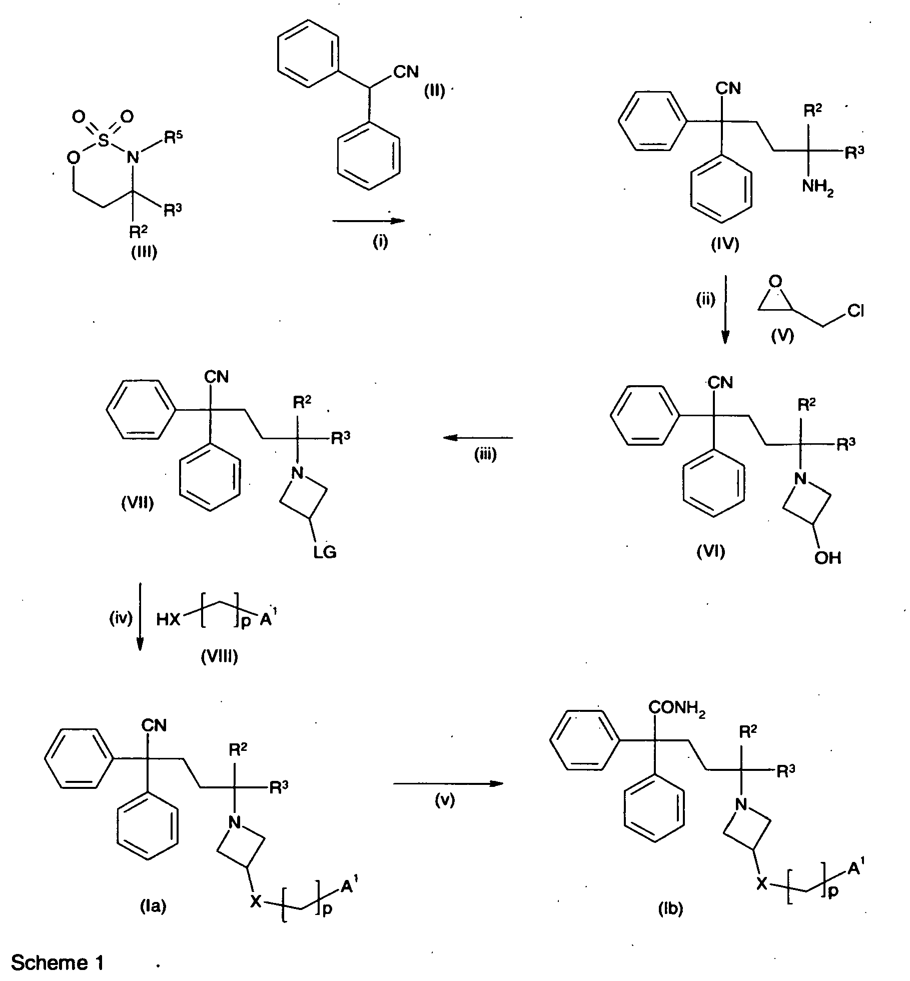 R⁵ is H or PG.
PG is a suitable protecting group.
R², R³, X, p and A¹ are as defined for compounds of formula (I).
LG represents a suitable leaving group such as mesylate or tosylate and is preferably mesylate.

Compounds of formula (III) may be prepared as described in WO2003037327, page 83, where PG represents a protecting group such as tert-butoxycarbonyl or benzyloxycarbonyl and is preferably tert-butoxycarbonyl. Alternatively, compounds of formula (III) may be prepared according to the following process:

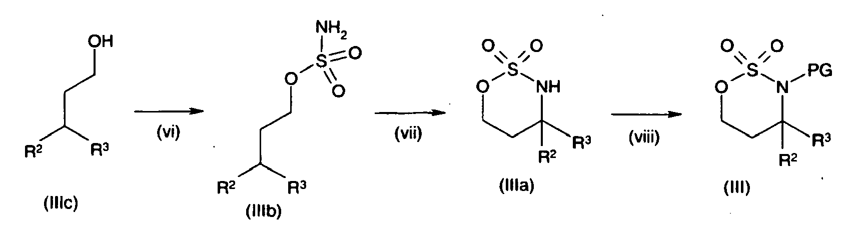

Compounds of formula (IIIc) are commercially available or known in the literature. Compounds of formula (IIIb) may be prepared from compounds of formula (IIIc) by process step (vi) - reaction of compounds (IIIc) with chlorosulfonyl isocyanate, formic acid and pyridine, in a suitable solvent such as dichloromethane, at low temperature for 2 hours. Typical conditions comprise 1.0 equivalent of compound (IIIc), 1.5. equivalents of chlorosulfonyl isocyanate, 1.5 equivalents of formic acid and 1.5 equivalents of pyridine in dichloromethane, at low temperature for 2 hours.
Compounds of formula (IIIa) may be prepared from compounds of formula (IIIb) by process step (vii) - reaction of compounds (IIIb) with magnesium oxide, iodobenzene diacetate and rhodium acetate dimer in a suitable solvent such as dichloromethane at room temperature for up to 24 hours. Typical conditions comprise reaction of 1.0 equivalent of compound (IIIb), 2.3 equivalent of magnesium dioxide, 1.1 equivalent of iodobenzene diacetate and 0.02 equivalent of rhodium acetate dimer in dichloromethane at room temperature for 18 hours. Compounds of formula (III) may be prepared from compounds of formula (IIIa) by incorporation of a suitable protecting group such as tert-butoxycarbonyl or benzyloxycarbonyl and is preferably tert-butoxycarbonyl, using conditions described in "Protecting Groups in Organic Synthesis" by T. W. Greene and P. Wutz. Typical conditions comprise reaction of 1.0 equivalent of compound (IIIa), 1.2 equivalents of di-tert-butyl dicarbonate, 2.0 equivalents of triethylamine and 0.2 equivalents of 4-dimethylaminopyridine in dichloromethane, at room temperature for 3 hours.

Compounds of formula (II) are commercially available.
Compounds of formula (IV) may be prepared from compounds of formula (II) and compounds of formula (III) by process step (i)-
1) Reaction of compounds (II) and (III) in the presence of a strong base such as potassium tert butoxide or sodium hydride, in a suitable solvent such as N,N-dimethylformamide or dimethylsulfoxide, under ambient conditions or at elevated temperature for up to 18 hours.
2) Removal of the protecting group (when used) using suitable conditions such as 4N hydrochloric acid in dioxan or trifluoroacetic acid or hydrogenation in the presence of catalytic palladium, as described in "Protecting Groups in Organic Synthesis" by T.W. Greene and P. Wutz.

Typical conditions comprise of 1.2 equivalents of compound (II), 1.0 equivalent of compound (III) and 1.2 equivalents of potassium tert butoxide in N,N-dimethylformamide, under ambient conditions for up to 18 hours, followed by treatment with 4N hydrochloric acid in dioxane.

Compounds of formula (V) are commercially available.

Compounds of formula (VI) may be prepared from compounds of formula (IV) and (V) by process step (ii)- heterocycle formation can be achieved by nucleophilic addition of compound (V) by compound (IV) followed by in situ ring closure, in a suitable solvent such as methanol or ethanol, at elevated temperature for up to 48 hours. Typical conditions comprise of 1.0 equivalent of compound (IV) and 1.1 equivalents of compound (V) in methanol, at elevated temperature for up to 48 hours.

Compounds of formula (VII) may be prepared from compounds of formula (VI) by process step (iii)- introduction of a suitable leaving group (LG), such as mesylate or tosylate groups by reaction of compound (VI) with mesyl chloride/anhydride or tosyl chloride, in the presence of a suitable base such as Hünig's base, triethylamine or pyridine, optionally in a suitable solvent such as dichloromethane or diethyl ether, at low temperature for 1-2 hours. Typical conditions comprise of 1.0 equivalent of compound (VI) and 3 equivalents of mesyl chloride in pyridine at low temperature for up to 1-2 hours.

Compounds of general formula (VIII) are commercially available, are known in the literature or they can be prepared easily by the man skilled in the art.

Compounds of formula (Ia) can be prepared from compounds of general formula (VII) and (VIII) by process step (iv)- optional treatment of compound (VIII) with a suitable base such caesium carbonate or sodium carbonate followed by reaction with compound (VII), in a suitable solvent such as N,N-dimethylformamide or dimethylsulfoxide, at elevated temperature for up to 18 hours. Typical conditions comprise of 1.0 equivalent of compound (VII), 3.0 equivalents of caesium carbonate and 3.0 equivalent of compound (VIII), in N,N-dimethylformamide, at elevated temperature for up to 18 hours.

In a further embodiment, compounds of formula (Ib) may be prepared from compounds of formula (Ia) by process step (v)- hydrolysis of compound (Ia) with an excess of potassium hydroxide in 3-methyl-3-pentanol, at elevated temperature for up to 24 hours. Typical conditions comprise of 1.0 equivalent of compound (Ia) and 20 equivalents of potassium hydroxide in 3-methyl-3-pentanol at elevated temperature for up to 24 hours.

Alternatively, compounds of formula (VI) may be prepared as described in scheme 2. 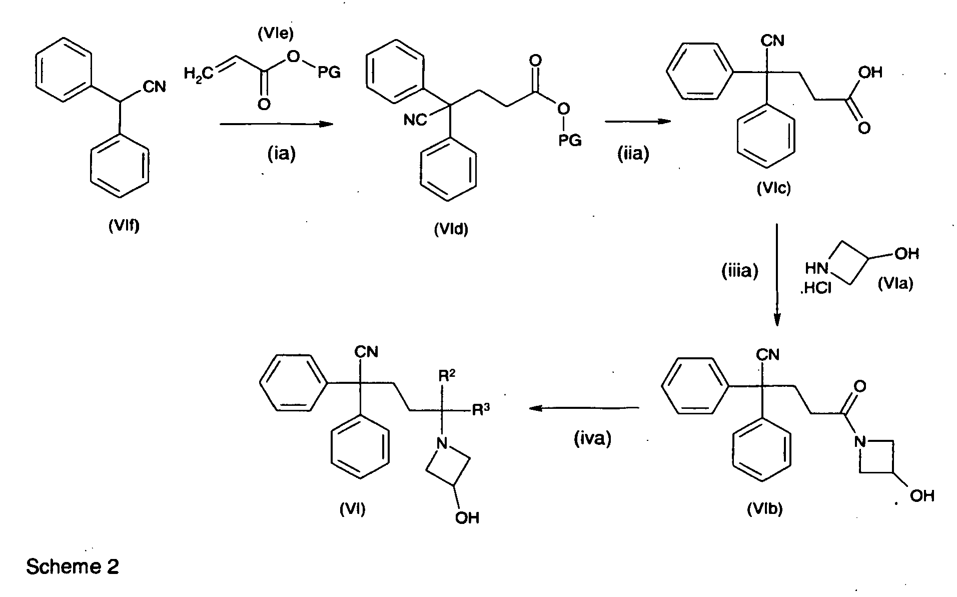 R² and R³ represent methyl.
PG is a suitable carboxyl-protecting group such as methyl or tert-butyl and is typically tert-butyl.

Compound of formula (Vlf) is commercially available.
Compounds of formula (VIe) are either commercially available or their preparation is known from the literature.
Compounds of formula (VId) may be prepared from compounds of formula (VIf) and (VIe) by process step (ia): compound (VIf) is treated with compound (VIe) in the presence of a suitable base such as potassium hydroxide or sodium hydroxide, in a suitable solvent such as methanol, ethanol or tert-butanol, at a temperature between 25°C and elevated temperature for 6-24 hours. Typical conditions comprise of 1.0 equivalent of compound (VIf), 0.05eq of potassium hydroxide and 1.0 equivalent of compound (VIe) in tert-butanol at a temperature between 25-60°C for up to 24 hours.
Compounds of formula (VIc) may be prepared from compounds of formula (VId) by process step (iia). De-protection of compound (VId) may be achieved using standard methodology as described in "Protecting Groups in Organic Synthesis" by T.W. Greene and P. Wutz. When PG is tert butyl, typical conditions comprise of 1.0 equivalent of compound (Vld) in the presence of hydrochloric acid (4M in dioxan) at room temperature for up to 18 hours.
Compound of formula (VIa) is commercially available.
Compounds of formula (VIb) may be prepared from compounds of formulae (VIc) and (VIa) by process step (iiia), coupling of (VIc) and (VIa) in the presence of a suitable coupling agent such as 1-(3-dimethylaminopropyl)-3-ethylcarbodiimide hydrochloride, N,N'-carbonyldiimidazole or N,N'-dicyclohexylcarbodiimide, optionally in the presence of a catalyst such as 1-hydroxybenzotriazole hydrate or 1-hydroxy-7-azabenzotriazole, and optionally in the presence of a tertiary amine base such as N-methylmorpholine, triethylamine or N,N-diisopropylethylamine, in a suitable solvent such as N,N-dimethylformamide, tetrahydrofuran or dichloromethane, under ambient conditions for 1-48 hours. Typical conditions comprise of 1.0 equivalent of compound (VIc), 1.0 equivalent of compound (Via) and 1.0-1.2 equivalents of 1-(3-dimethylaminopropyl)-3-ethylcarbodiimide hydrochloride, 1.0-1.2 equivalents of 1-hydroxybenzotriazole hydrate and 1.0-2.0 equivalents of triethylamine in dichloromethane, at room temperature for 18 hours.

Compounds of formula (VI) can be prepared from compound of formula (VIb) in analogy to the methods of Denton and Wood (Synlett, 1999, 1, 55); Compound (VIb) is typically preactivated with a suitable Lewis acid such as titanium (IV) chloride or zirconium (IV) chloride then treated with an excess of a suitable organometallic reagent such as MeMgCl or MeMgBr, in a suitable solvent such as tetrahydrofuran or diethyl ether, at a temperature between -78°C to 25°C, for 1-18 hours. Typical conditions comprise of 1.0 equivalent of compound (Vlb), 2 equivalents of zirconium (IV) chloride and 9.0 equivalents of MeMgCl in tetrahydrofuran, at - 30°C for 4-8 hours.

Alternatively compounds of formula (I) may be prepared as described in scheme 3.

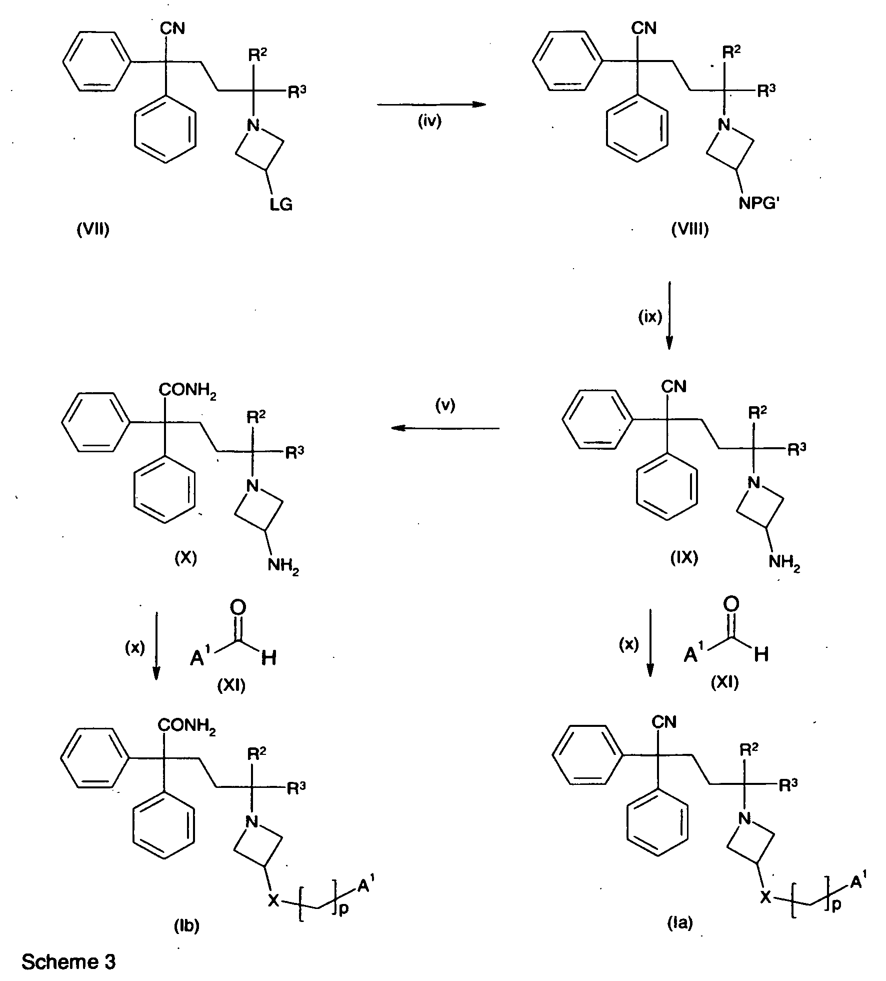

LG represents a suitable leaving group such as mesylate or tosylate and is preferably mesylate.
X is NH and p is 1.
PG' represents a suitable amine protecting group such as phthalimide or benzyl and is preferably phthalimide.
Compounds of formula (VII) are prepared as described in scheme 1. Compounds of formula (VIII) may be prepared from compounds of formula (VII) by reaction with ammonia suitably protected with PG', under process step (iv). When PG' is phthalimide, typical conditions comprise reaction of 1.0 equivalent of compound (VII) with 1.0 equivalent of phthalimide and 2.0 equivalents of a suitable base such as cesium carbonate, in a suitable solvent such as dimethylformamide at elevated temperature for 2hrs.

Compounds of formula (IX) are prepared from compounds of formula (VIII) by removal of the protecting group, by process step (ix), using suitable conditions such as hydrazine hydrate or hydrogenation in the presence of catalytic palladium, as described in "Protecting Groups in Organic Synthesis" by T.W. Greene and P. Wutz. When PG' is phthalimide, typical conditions comprise reaction of 1.0 equivalent of compound (VIII) with 10.0 equivalents of hydrazine hydrate in a suitable solvent such as ethanol at elevated temperature for 1 hr.
Compounds of formula (X) are prepared from compounds of formula (IX) by process step (v), as described in scheme 1.
Compounds of formula (XI) are either commercially available or known in the literature.
Compounds of formula (Ib) are prepared from compounds of formula (X) and compounds of formula (XI) by process step (x) - reductive amination using a suitable reducing agent such as sodium triacetoxyborohydride in the presence of acid such as acetic acid, in a suitable solvent such as dichloromethane. Typical conditions comprise reaction of 1.0 equivalent of compound (X) with 2 equivalents of compound (XI) and 1 drop of acetic acid in dichloromethane at room temperature for 1 hour followed by addition of 2 equivalents of sodium triacetoxyborohydride and reaction at room temperature for a further 18 hours.
In further examples, compounds of formula (Ia) are prepared from compounds of formula (IX) and compounds of formula (XI) by process step (x).
In further examples of formula (I), where A¹ represents an optionally substituted methoxyphenyl, it may be desirable to de-alkylate the substrate to provide the corresponding phenol. Typical conditions of this procedure comprise of 1.0 equivalent of compound (I) and 1-4 equivalents of 1M boron tribromide in dichloromethane, in a suitable solvent such as dichloromethane, at low temperature for 1-18 hours.

Pharmaceutically acceptable salts of the compounds of formula (I) include the acid addition and base salts thereof.
Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

For a review on suitable salts, see Handbook of Pharmaceutical Salts: Properties, Selection, and Use by Stahl and Wermuth (Wiley-VCH, 2002).

Pharmaceutically acceptable salts of compounds of formula (I) may be prepared by one or more of three methods:
(i) by reacting the compound of formula (I) with the desired acid or base;
(ii) by removing an acid- or base-labile protecting group from a suitable precursor of the compound of formula (I) or by ring-opening a suitable cyclic precursor, for example, a lactone or lactam, using the desired acid or base; or
(iii) by converting one salt of the compound of formula (I) to another by reaction with an appropriate acid or base or by means of a suitable ion exchange column.
All three reactions are typically carried out in solution. The resulting salt may precipitate out and be collected by filtration or may be recovered by evaporation of the solvent. The degree of ionisation in the resulting salt may vary from completely ionised to almost non-ionised.

The compounds of the invention may exist in a continuum of solid states ranging from fully amorphous to fully crystalline. The term 'amorphous' refers to a state in which the material lacks long range order at the molecular level and, depending upon temperature, may exhibit the physical properties of a solid or a liquid. Typically such materials do not give distinctive X-ray diffraction patterns and, while exhibiting the properties of a solid, are more formally described as a liquid. Upon heating, a change from solid to liquid properties occurs which is characterised by a change of state, typically second order ('glass transition'). The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterised by a phase change, typically first order ('melting point').

The compounds of the invention may also exist in unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water.

A currently accepted classification system for organic hydrates is one that defines isolated site, channel, or metal-ion coordinated hydrates - see Polymorphism in Pharmaceutical Solids by K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

The compounds of the invention may also exist as multi-component complexes (other than salts and solvates) wherein the drug and at least one other component are present in stoichiometric or non-stoichiometric amounts. Complexes of this type include clathrates (drug-host inclusion complexes) and co-crystals. The latter are typically defined as crystalline complexes of neutral molecular constituents which are bound together through non-covalent interactions, but could also be a complex of a neutral molecule with a saft. Co-crystals may be prepared by melt crystallisation, by recrystallisation from solvents, or by physically grinding the components together - see Chem Commun, 17, 1889-1896, by O. Almarsson and M. J. Zaworotko (2004). For a general review of multi-component complexes, see J Pharm Sci, 64 (8), 1269-1288, by Haleblian (August 1975).

The compounds of the invention may also exist in a mesomorphic state (mesophase or liquid crystal) when subjected to suitable conditions. The mesomorphic state is intermediate between the true crystalline state and the true liquid state (either melt or solution). Mesomorphism arising as the result of a change in temperature is described as 'thermotropic' and that resulting from the addition of a second component, such as water or another solvent, is described as `lyotropic'. Compounds that have the potential to form lyotropic mesophases are described as 'amphiphilic' and consist of molecules which possess an ionic (such as - COO⁻Na⁺, -COO⁻K⁺, or -SO₃⁻Na⁺) or non-ionic (such as -N⁻N⁺(CH₃)₃) polar head group. For more information, see Crystals and the Polarizing Microscope by N. H. Hartshorne and A. Stuart, 4th Edition (Edward Arnold, 1970).

Hereinafter all references to compounds of formula (I) include references to salts, solvates, multi-component complexes and liquid crystals thereof and to solvates, multi-component complexes and liquid crystals of salts thereof.

Moreover, certain compounds of formula (I) may themselves act as prodrugs of other compounds of formula I.

Also included within the scope of the invention are metabolites of compounds of formula I, that is, compounds formed *in vivo* upon administration of the drug. Some examples of metabolites in accordance with the invention include
(i) where the compound of formula (I) contains a methyl group, an hydroxymethyl derivative thereof (-CH₃ -> -CH₂OH):
(ii) where the compound of formula (I) contains an alkoxy group, an hydroxy derivative thereof (-OR -> -OH);
(iii) where the compound of formula (I) contains a tertiary amino group, a secondary amino derivative thereof (-NR¹R² -> -NHR¹ or -NHR²);
(iv) where the compound of formula (I) contains a secondary amino group, a primary derivative thereof (-NHR¹ -> -NH₂);
(v) where the compound of formula (I) contains a phenyl moiety, a phenol derivative thereof (-Ph -> -PhOH); and
(vi) where the compound of formula (I) contains an amide group, a carboxylic acid derivative thereof (-CONH₂ -> COOH).

Compounds of formula (I) containing one or more asymmetric carbon atoms can exist as two or more stereoisomers. Where a compound of formula (I) contains an alkenyl or alkenylene group, geometric *cis*/*trans* (or Z/E) isomers are possible. Where structural isomers are interconvertible *via* a low energy barrier, tautomeric isomerism ('tautomerism') can occur. This can take the form of proton tautomerism in compounds of formula (I) containing, for example, an imino, keto, or oxime group, or so-called valence tautomerism in compounds which contain an aromatic moiety. It follows that a single compound may exhibit more than one type of isomerism.
Included within the scope of the present invention are all stereoisomers, geometric isomers and tautomeric forms of the compounds of formula I, including compounds exhibiting more than one type of isomerism, and mixtures of one or more thereof. Also included are acid addition or base salts wherein the counterion is optically active, for example, *d*-lactate or /lysine, or racemic, for example, *dl*-tartrate or *dl*-arginine.

*Cis*/*trans* isomers may be separated by conventional techniques well known to those skilled in the art, for example, chromatography and fractional crystallisation.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (I) contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. Concentration of the eluate affords the enriched mixture.
When any racemate crystallises, crystals of two different types are possible. The first type is the racemic compound (true racemate) referred to above wherein one homogeneous form of crystal is produced containing both enantiomers in equimolar amounts. The second type is the racemic mixture or conglomerate wherein two forms of crystal are produced in equimolar amounts each comprising a single enantiomer.

While both of the crystal forms present in a racemic mixture have identical physical properties, they may have different physical properties compared to the true racemate. Racemic mixtures may be separated by conventional techniques known to those skilled in the art - see, for example, Stereochemistry of Organic Compounds by E. L. Eliel and S. H. Wilen (Wiley, 1994).

The present invention includes all pharmaceutically acceptable isotopically-labelled compounds of formula (I) wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of formula I, for example, those incorporating a radioactive isotope, are useful in drug and/or substrate tissue distribution studies. The radioactive isotopes tritium, *i.e*. ³H, and carbon-14, *i.e*. ¹⁴C , are particularly useful for this purpose in view of their ease of incorporation and ready means of detection.

Substitution with heavier isotopes such as deuterium, *i.e*. ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of formula (I) can generally be prepared by conventional techniques known to those skilled in the art or by processes analogous to those described in the accompanying Examples and Preparations using an appropriate isotopically-labeled reagent in place of the non-labeled reagent previously employed.

Pharmaceutically acceptable solvates in accordance with the invention include those wherein the solvent of crystallization may be isotopically substituted, *e.g*. D₂O, d₆-acetone, d₆-DMSO.

The compounds of formula (I) should be assessed for their biopharmaceutical properties, such as solubility and solution stability (across pH), permeability, *etc*., in order to select the most appropriate dosage form and route of administration for treatment of the proposed indication.

Compounds of the invention intended for pharmaceutical use may be administered as crystalline or amorphous products. They may be obtained, for example, as solid plugs, powders, or films by methods such as precipitation, crystallization, freeze drying, spray drying, or evaporative drying. Microwave or radio frequency drying may be used for this purpose.

They may be administered alone or in combination with one or more other compounds of the invention or in combination with one or more other drugs (or as any combination thereof). Generally, they will be administered as a formulation in association with one or more pharmaceutically acceptable excipients. The term 'excipient' is used herein to describe any ingredient other than the compound(s) of the invention. The choice of excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in Remington's Pharmaceutical Sciences, 19th Edition (Mack Publishing Company, 1995).

The compounds of the invention may be administered orally. Oral administration may involve swallowing, so that the compound enters the gastrointestinal tract, and/or buccal, lingual, or sublingual administration by which the compound enters the blood stream directly from the mouth.

Formulations suitable for oral administration include solid, semi-solid and liquid systems such as tablets; soft or hard capsules containing multi- or nano-particulates, liquids, or powders; lozenges (including liquid-filled); chews; gels; fast dispersing dosage forms; films; ovules; sprays; and buccal/mucoadhesive patches.

Liquid formulations include suspensions, solutions, syrups and elixirs. Such formulations may be employed as fillers in soft or hard capsules (made, for example, from gelatin or hydroxypropylmethylcellulose) and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1, by H. Lieberman and L. Lachman (Marcel Dekker, New York, 1980).

Consumable oral films for human or veterinary use are typically pliable water-soluble or water-swellable thin film dosage forms which may be rapidly dissolving or mucoadhesive and typically comprise a compound of formula I, a film-forming polymer, a binder, a solvent, a humectant, a plasticiser, a stabiliser or emulsifier, a viscosity-modifying agent and a solvent. Some components of the formulation may perform more than one function.

The compound of formula (I) may be water-soluble or insoluble. A water-soluble compound typically comprises from 1 weight % to 80 weight %, more typically from 20 weight % to 50 weight %, of the solutes. Less soluble compounds may comprise a greater proportion of the composition, typically up to 88 weight % of the solutes: Alternatively, the compound of formula (I) may be in the form of multiparticulate beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Pharmaceutical Technology On-line, 25(2), 1-14, by Verma et al (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular, intrasynovial and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplishes using standard pharmaceutical techniques well known to those skilled in the art.

The solubility of compounds of formula (I) used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a suspension or as a solid, semi-solid, or thixotropic liquid for administration as an implanted depot providing modified release of the active compound. Examples of such formulations include drug-coated stents and semi-solids and suspensions comprising drug-loaded poly(*dl*-lactic-coglycolic)acid (PGLA) microspheres.

The compounds of the invention may also be administered- topically, (intra)dermally, or transdermally to the skin or mucosa. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999).

Other means of topical administration include delivery by electroporation, iontophoresis, phonophoresis, sonophoresis and microneedle or needle-free (*e.g*. Powderject^{™}, Bioject^{™}, *etc*.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler, as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane, or as nasal drops. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as I-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1 µg to 20mg of the compound of the invention per actuation and the actuation volume may vary from 1µl to 100µl. A typical formulation may comprise a compound of formula I, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing from 0.001mg to 10mg of the compound of formula (I). The overall daily dose will typically be in the range 0.001 mg to 40mg which may be administered in a single dose or, more usually, as divided doses throughout the day.

The compounds of formula (I) are particularly suitable for an administration by inhalation

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, gels, biodegradable (*e.g*. absorbable gel sponges, collagen) and non-biodegradable (*e.g*. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis. Formulations for ocular/aural administration may be formulated to be immediate and/or modified release. Modified release formulations include delayed-,sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, *i.e*. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

Inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Thus the kit of the invention comprises two or more separate pharmaceutical compositions, at least one of which contains a compound of formula (I) in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

For administration to human patients, the total daily dose of the compounds of the invention is typically in the range 0.001 mg to 5000mg depending, of course, on the mode of administration. For example, oral administration may require a total daily dose of from 0.1 mg to 1000mg, while an intravenous dose may only require from 0.001 mg to 100mg. The total daily dose may be administered in single or divided doses and may, at the physician's discretion, fall outside of the typical range given herein.

These dosages are based on an average human subject having a weight of about 60kg to 70kg. The physician will readily be able to determine doses for subjects whose weight falls outside this range, such as infants and the elderly.
For the avoidance of doubt, references herein to "treatment" include references to curative, palliative and prophylactic treatment.
The compounds of formula (I) have the ability to interact with muscarinic receptors and thereby have a wide range of therapeutic applications, as described further below, because of the essential role which muscarinic receptors play in the physiology of all mammals.
Thus the invention relates to the use of the compounds of formula (I) for the manufacture of a medicament for the treatment or the prevention of diseases, disorders, and conditions in which the M3 receptor is involved. The invention further relates to a method of treatment of a mammal, including a human being, with a M3 antagonist including treating said mammal with an effective amount of a compound of the formula (I) or with a pharmaceutically acceptable salt, derived form or composition thereof.

Therefore, a further aspect of the present invention relates to the compounds of formula (I), or pharmaceutically acceptable salts, derived forms or compositions thereof, for use in the treatment of diseases, disorders, and conditions in which muscarinic receptors are involved. Examples of such diseases, disorders, and conditions are Inflammatory Bowel Disease, Irritable Bowel Disease, diverticular disease, motion sickness, gastric ulcers, radiological examination of the bowel, symptomatic treatment of BPH (benign prostatic hyperplasia), NSAID induced gastric ulceration, urinary Incontinence (including urgency, frequency, urge incontinence, overactive bladder, nocturia and Lower urinary tract symptoms), cycloplegia, mydriatics, parkinsons disease.

More specifically, the present invention also concerns the compounds of formula (I), or pharmaceutically acceptable salts, derived forms or compositions thereof, for use in the treatment of diseases, disorders, and conditions selected from the group consisting of :
- chronic or acute bronchoconstriction, chronic bronchitis, small airways obstruction, and emphysema,
- obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated or not associated with COPD, COPD that is characterized by irreversible, progressive airways obstruction, adult respiratory distress syndrome (ARDS), exacerbation of airways hyper-reactivity consequent to other drug therapy and airways disease that is associated with pulmonary hypertension,
- bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, • infectious asthmatic bronchitis, productive bronchitis, staphylococcus or streptococcal bronchitis and vesicular bronchitis,
- asthma of whatever type, etiology, or pathogenesis, in particular asthma that is a member selected from the group consisting of atopic asthma, non-atopic asthma, allergic asthma, atopic bronchial IgE-mediated asthma, bronchial asthma, essential asthma, true asthma, intrinsic asthma caused by pathophysiologic disturbances, extrinsic asthma caused by environmental factors, essential asthma of unknown or inapparent cause, non-atopic asthma, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma, wheezy infant syndrome and bronchiolytis,
- acute lung injury,
- bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis.

More specifically, the present invention also concerns the compounds of formula (I), or pharmaceutically acceptable salts, derived forms or compositions thereof, for use in the treatment of COPD or asthma.

Suitable examples of other therapeutic agents which may be used in combination with the compound(s) of formula (I), or pharmaceutically acceptable salts, derived forms or compositions thereof, include, but are by no means limited to :
(a) 5-Lipoxygenase (5-LO) inhibitors or 5-lipoxygenase activating protein (FLAP) antagonists,
(b) Leukotriene antagonists (LTRAs) including antagonists of LTB₄, LTC₄, LTD₄, and LTE₄,
(c) Histamine receptor antagonists including H1 and H3 antagonists,
(d) α₁- and α₂-adrenoceptor agonist vasoconstrictor sympathomimetic agents for decongestant use,
(e) short or long acting β₂ agonists,
(f) PDE inhibitors, e.g. PDE3, PDE4 and PDE5 inhibitors,
(g) Theophylline,
(h) Sodium cromoglycate,
(i) COX inhibitors both non-selective and selective COX-1 or COX-2 inhibitors (NSAIDs),
(j) Oral and inhaled glucocorticosteroids,
(k) Monoclonal antibodies active against endogenous inflammatory entities,
(l) Anti-tumor necrosis factor (anti-TNF-α) agents,
(m) Adhesion molecule inhibitors including VLA-4 antagonists,
(n) Kinin-B₁ - and B₂-receptor antagonists,
(o) Immunosuppressive agents,
(p) Inhibitors of matrix metalloproteases (MMPs),
(q) Tachykinin NK₁, NK₂ and NK₃ receptor antagonists,
(r) Elastase inhibitors,
(s) Adenosine A2a receptor agonists,
(t) Inhibitors of urokinase,
(u) Compounds that act on dopamine receptors, e.g. D2 agonists,
(v) Modulators of the NFκB pathway, e.g. IKK inhibitors,
(w) modulators of cytokine signalling pathyways such as p38 MAP kinase, syk kinase, or JAK kinase inhibitors,
(x) Agents that can be classed as mucolytics or anti-tussive,
(y) Antibiotics,
(z) Prostaglandin antagonists such as DP1, DP2 or CRTH2 antagonists,
(aa) HDAC inhibitors,
(bb)PI3 kinase inhibitors, and,
(cc) CXCR2 antagonists.

According to the present invention, combination of the compounds of formula (I) with :
- H3 antagonists,
- β₂ agonists,
- PDE4 inhibitors,
- steroids, especially glucocorticosteroids,
- Adenosine A2a receptor agonists,
- Modulators of cytokine signalling pathyways such as p38 MAP kinase or syk kinase, or,
- Leukotriene antagonists (LTRAs) including antagonists of LTB₄, LTC₄, LTD₄, and LTE₄,
are preferred.

According to the present invention, combination of the compounds of formula (I) with :
- glucocorticosteroids, in particular inhaled glucocorticosteroids with reduced systemic side effects, including prednisone, prednisolone, flunisolide, triamcinolone acetonide, beclomethasone dipropionate, budesonide, fluticasone propionate, ciclesonide, and mometasone furoate, or
- β2 agonists including in particular salbutamol, terbutaline, bambuterol, fenoterol, salmeterol, formoterol, tulobuterol and their salts. are further preferred.

The following examples illustrate the preparation of the compounds of the formula (I):

### Reparation 1

### 5-Amino-5-methyl-2,2-diphenylhexanenitrile

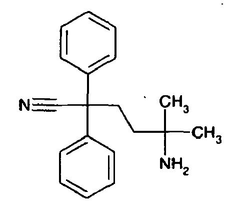

Potassium tert-butoxide (203mg, 1.81mmol) and tert-butyl 4,4-dimethyl-1,2,3-oxathiazinane-3-carboxylate 2,2-dioxide[(400mg, 1.51mmol), WO2003037327, p83] were added to a solution of diphenylacetonitrile (349mg, 1.81mmol) in N,N-dimethylformamide (5mL) and the mixture was stirred for 18 hours at room temperature. The reaction mixture was then concentrated in vacuo and the residue was treated with hydrochloric acid (4M in dioxane, 10mL) and heated at 40°C for 2.5 hours. The reaction mixture was concentrated in vacuo and the residue was basified with saturated sodium hydrogen carbonate solution and extracted with ethyl acetate (2x30mL). The combined organic solution was dried over magnesium sulfate, concentrated in vacuo and the residue was purified by column chromatography on silica gel, eluting with dichloromethane:methanol:0.88 ammonia, 90:10:1, to afford the title compound as a colourless oil in 77% yield, 324mg.
¹HNMR(400MHz, CDCl₃) δ: 1.17(m, 6H), 1.48-1.57(m, 2H), 2.20-2.40(brs, 2H), 2.42-2.53(m, 2H), 7.22-7.43(m, 10H); LRMS APCI m/z 279 [M+H]⁺

### Preparation 2

### 5-(3-Hydroxyazetidin-1-yl)-5-methyl-2,2-diphenylhexanenitrile

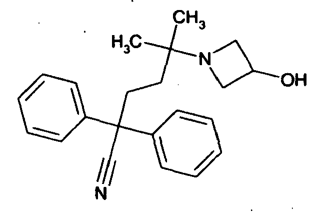

A mixture of (+/-)-epichlorohydrin (1.47mL, 18.76mmol) and the product of preparation 1 (4.74g, 17mmol) in methanol (50mL) was heated at 60°C for 48 hours. The reaction mixture was then concentrated in vacuo and the residue was partitioned between ethyl acetate (50mL) and sodium hydrogen carbonate solution (30mL). The aqueous layer was separated and extracted with ethyl acetate (2x50mL). The combined organic solution was dried over magnesium sulfate, concentrated in vacuo and the residue was purified by column chromatography on silica gel, eluting with dichloromethane:methanol:0.88 ammonia, 100:0:0 to 95:5:0.5, to afford the title compound as a pale yellow oil in 50% yield, 2.86g.
¹HNMR(400MHz, CDCl₃) δ: 0.93(s, 6H), 1.29-1.39(m, 2H), 2.38-2.50(m, 2H), 2.90-3.00(m, 2H), 3.29-3.39(m, 2H), 4.29-4.39(m, 1H), 7.24-7.45(m, 10H); LRMS APCI m/z 335 [M+H]⁺

### Preparation 3

### 1-(4-Cyano-1,1-dimethyl-4,4-diphenylbutyl)azetidin-3-yl methanesulfonate

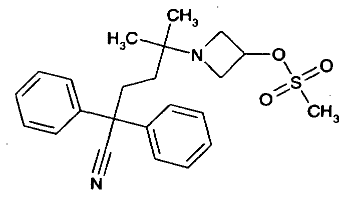

Methane sulfonyl chloride (3.3mL, 43mmol) was added to a solution of the product of preparation 2 (4.82g, 14.4mmol) in pyridine (50mL), cooled to -15°C. The mixture was stirred for 2 hours, allowing the temperature to warm to 0°C, then concentrated in vacuo. The residue was partitioned between ethyl acetate (100mL) and sodium hydrogen carbonate solution (100mL) and the organic layer was separated, dried over magnesium sulfate and concentrated in vacuo. Purification of the residue by column chromatography on silica gel, eluting with pentane:ethylacetate/methanol/0.88 ammonia (90/10/1) 2:1, afforded the title compound as a yellow oil in 81% yield, 4.80g.
¹HNMR(400MHz, CDCl₃) δ: 0.95(s, 6H), 1.30-1.41(m, 2H), 2.42-2.55(m, 2H), 2.98(s, 3H), 3.25-3.37(m, 2H), 3.44-3.56(m, 2H), 5.00-5.06(m, 1H), 7.23-7.44(m, 10H); LRMS APCI m/z 413 [M+H]⁺

### Preparation 4

### 5-[3-(1,3-Dioxo-1,3-dihydro-isoindol-2-yl)-azetidin-1-yl]-5-methyl-2,2-diphenyl-hexanenitrile

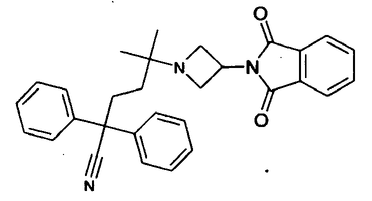

The product of preparation 3 (2.5g, 6.1 mmol) was dissolved in N,N-dimethylformamide (20mL) and phthalimide (1.1g, 2.6 mmol) and cesium carbonate (3.9g, 12mmol) were added. The reaction mixture was stirred at 80°C for 2 hours then concentrated in vacuo. The residue was diluted with saturated sodium hydrogen carbonate solution (50mL) and extracted with ethyl acetate (2x50mL). The combined organic solution was dried over magnesium sulphate and concentrated in vacuo. The product was used in the next reaction without further purification. LRMS ESI m/z 464 [M+H]⁺

### Preparation 5

### 5-(3-Amino-azetidin-1-yl)-5-methyl-2,2-diphenyl-hexanenitrile

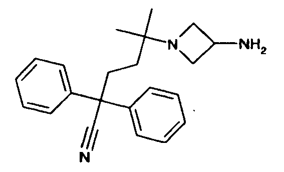

The product of preparation 4 (-6.1 mmol) was dissolved in ethanol (50mL) and hydrazine hydrate (3.0g, 60mmol) was added. The reaction mixture was stirred at 60°C for 1 hour then concentrated in vacuo. The residue was diluted with saturated sodium hydrogen carbonate solution (50mL) and extracted with ethyl acetate (50mL). The organic solution was dried over magnesium sulphate and concentrated in vacuo. Purification of the residue by column chromatography on silica gel, eluting with dichloromethane:methanol:0.88 ammonia, 95:5:0.5 to 90:10:1, afforded the title compound as a colourless oil in 86% yield, 1.73g.
¹HNMR(400MHz, DMSO) δ: 0.82 (s, 6H), 1.12-1.19 (m, 2H), 2.40-2.47 (m, 2H), 2.57-2.65 (m, 2H), 3.07-3.15 (m, 2H), 3.20-3.32 (m, 1H), 7.25-7.42 (m, 10H); LRMS ESI m/z 334 [M+H]⁺

### Preparation 6

### 5-(3-Amino-azetidin-1-yl)-5-methyl-2,2-diphenyl-hexanoic acid amide

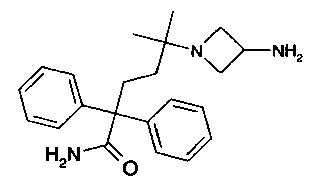

The product of preparation 5 (1.24g, 3.72 mmol) was dissolved in 3-methyl-3-pentanol (20mL) and powdered potassium hydroxide (4.2g, 75mmol) added. The reaction mixture was stirred at 120°C for 18 hours then concentrated in vacuo. The residue was diluted with water (40mL) and extracted with ethyl acetate (2x50mL). The combined organics were dried over magnesium sulphate and concentrated in vacuo. Purification of the residue by column chromatography on silica gel, eluting with dichloromethane:methanol:0.88 ammonia, 90:10:1 to 80:20:2, afforded the title compound as a colourless oil in 86% yield, 1.12g.
¹HNMR(400MHz, CDCl3) δ: 0.90 (s, 6H), 1.09-1.18 (m, 2H), 2.38-2.46 (m, 2H), 2.72-2.80 (m, 2H), 3.34-3.40 (m, 2H), 3.43-3.56 (m, 1H), 5.50-5.64 (br m, 2H), 7.19-7.38 (m, 10H); LRMS ESI m/z 352 [M+H]⁺

### Example 1

### 5-(3-Benzylamino-azetidin-1-yl)-5-methyl-2,2-diphenyl-hexanenitrile

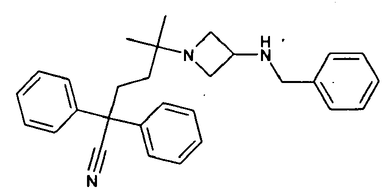

The product of preparation 3 (96mg, 0.23 mmol) was dissolved in N,N-dimethylformamide (3mL) and benzylamine (50µL, 0.46mmol) added. The reaction mixture was stirred at 70°C for 3 hours then concentrated in vacuo. Purification of the residue by column chromatography on silica gel, eluting with pentane:ethylacetate/methanol/0.88 ammonia (90/10/1) 95:5 to 50:50, afforded the title compound as a colourless oil in 24% yield, 24mg.
¹HNMR(400MHz, CDCl₃) δ: 0.93 (s, 6H), 1.28-1.37 (m, 2H), 2.40-2.52 (m, 2H), 2.73-2.84 (m, 2H), 3.26-3.35 (m, 2H), 3.36-3.46 (m, 1H), 3.72 (s, 2H), 7.22-7.43 (m, 15H); LRMS APCI m/z 424 [M+H]⁺

### Example 2

### 5-(3-Benzylamino-azetidin-1-yl)-5-methyl-2,2-diphenyl-hexanoic acid amide

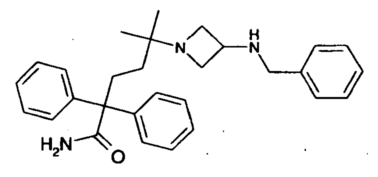

The product of example 1 (24mg, 0.057 mmol) was dissolved in 3-methyl-3-pentanol (5mL) and powdered potassium hydroxide added (64mg, 1.1mmol). The reaction mixture was stirred at 120°C for 18 hours then further potassium hydroxide was added (50mg, 0.89mmol). After a stirring for 6 hours at 120°C, the reaction mixture was concentrated in vacuo. The residue was diluted with water (20mL) and extracted with ethyl acetate (2x20mL). The combined organics were dried over magnesium sulphate and concentrated in vacuo. Purification of the residue by column chromatography on silica gel, eluting with pentane:ethylacetate/methanol/0.88 ammonia (90/10/1) 95:5 to 50:50, afforded the title compound as a colourless oil in 92% yield, 23mg.
¹HNMR(400MHz, CDCl3) δ: 0.88 (s, 6H), 1.10-1.18 (m, 2H), 2.38-2.46 (m, 2H), 2.74-2.83 (m, 2H), 3.26-3.35 (m, 2H), 3.36-3.45 (m, 1H), 3.70 (s, 2H), 5.40-5.60 (br m, 2H), 7.20-7.40 (m, 15H); LRMS APCI m/z 442 [M+H]⁺

### Example 3

### 5-[3-(2-Chloro-3-hydroxybenzylamino)-azetidin-1-yl]-5-methyl-2,2-diphenyl-hexanoic acid amide

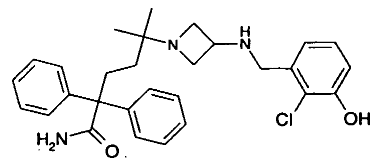

The product of preparation 6 (47mg, 0.13 mmol) was dissolved in dichloromethane (5mL) and 2-chloro-3-hydroxybenzaldehyde (42mg, 0.27mmol) was added. One drop of glacial acetic acid was added and the reaction mixture was stirred at room temperature for 1 hour. Sodium triacetoxyborohydride (57mg, 0.27mmol) was added and reaction mixture stirred at room temperature for 18 hours. The reaction mixture was washed with saturated sodium hydrogen carbonate solution (20mL) and the organic layer was dried over magnesium sulphate and concentrated in vacuo. Purification of the residue by column chromatography on silica gel, eluting with pentane:ethylacetate/methanol/0.88 ammonia (90/10/1) 66:33 to 0:100, afforded the title compound as a colourless foam in 27% yield, 18mg.
¹HNMR(400MHz, CDCl3) δ: 0.95(s, 6H), 1.11-1.22 (m, 2H), 2.40-2.48 (m, 2H), 2.80-3.00 (m, 2H), 3.33-3.52 (m, 3H), 3.78 (s, 2H), 5.48-5.64 (br m, 2H), 6.82-6.95 (m, 2H), 7.04-7.13 (m, 1H), 7.20-7.38 (m, 10H); LRMS ESI m/z 492 [M+H]⁺

### Example 4

### 5-[3-(5-Chloro-2-hydroxy-benzylamino)-azetidin-1-yl]-5-methyl-2,2-diphenyl-hexanoic acid amide

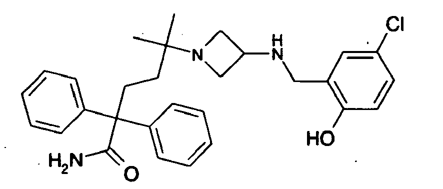

The title compound was prepared from the product of preparation 6 and 5-chloro-2-hydroxybenzaldehyde using a similar method to that described for example 3, as a colourless oil in 50%.yield. ¹HNMR(400MHz, CDCl3) δ: 0.92 (s, 6H), 1.13-1.20 (m, 2H), 2.40-2.48 (m, 2H), 2.85-3.04 (m, 2H), 3.30-3.40 (m, 3H), 3.83 (s, 2H), 5.43-5.60 (br m, 2H), 6.69-6.75 (m, 1H), 6.92 (s, 1H), 7.06-7.12 (m, 1H), 7.23-7.38 (m, 10H); LRMS ESI m/z 492 [M+H]⁺

### Example 5

### 5-[3-(5-Fluoro-2-hydroxy-benzylamino)-azetidin-1-yl]-5-methyl-2,2-diphenyl-hexanoic acid amide

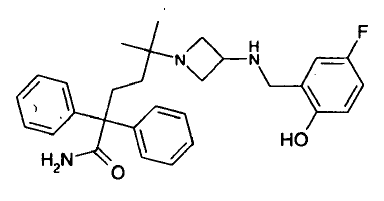

The title compound was prepared from the product of preparation 6 and 5-fluoro-2-hydroxybenzaldehyde using a similar method to that described for example 3, as a colourless oil in 37% yield. ¹HNMR(400MHz, MeOD) δ: 1.27 (s, 6H), 1.37-1.43 (m, 2H), 2.42-2.54 (m, 2H), 4.18-4.38 (m, 7H), 6.88-6.75 (m, 1H), 7.05-7.12 (m, 1H), 7.13-7.19 (m, 1H), 7.25-7.40 (m, 10H); LRMS ESI m/z 476 [M+H]⁺ .

### Example 6

### 5-[3-(3-Hydroxy-benzylamino)-azetidin-1-yl]-5-methyl-2,2-diphenyl-hexanoic acid amide

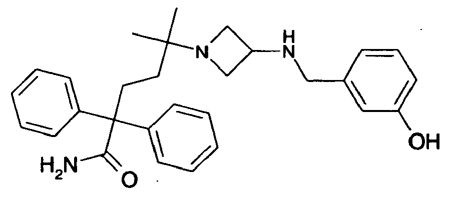

The title compound was prepared from the product of preparation 6 and 3-hydroxybenzaldehyde using a similar method to that described for example 3, as a colourless oil in 54%.yield. ¹HNMR(400MHz, MeOD) δ: 0.95(s, 6H), 1.06-1.15 (m, 2H), 2.30-2.39 (m, 2H), 2.95-3.03 (m, 2H), 3.22-3.38 (m, 3H), 3.59 (s, 2H), 6.64-6.78 (m, 3H), 7.08-7.15 (m, 1H), 7.22-7.40 (m, 10H); LRMS ESI m/z 458 [M+H]⁺

### Example 7

### 5-[3-(5-Fluoro-2-hydroxy-benzylamino)-azetidin-1-yl]-5-methyl-2,2-diphenyl-hexanenitrile

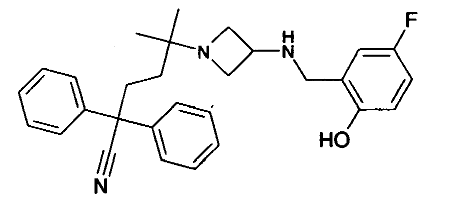

The title compound was prepared from the product of preparation 5 and 5-fluoro-2-hydroxybenzaldehyde using a similar method to that described for example 3, as a colourless oil in 63% yield. ¹HNMR(400MHz, CDCl3) δ: 0.90 (s, 6H), 1.25-1.38 (m, 2H), 2.40-2.53 (m, 2H), 2.82-2.95 (m, 2H), 3.25-3.42 (m, 3H), 3.85 (s, 2H), 6.64-6.73 (m, 1H), 6.74-6.80 (m, 1H), 6.81-6.92 (m, 1H), 7.23-7.43 (m, 10H); LRMS ESI m/z 458 [M+H]⁺

### Example 8

### 5-[3-(5-Chloro-2-hydroxy-benzylamino)-azetidin-1-yl]-5-methyl-2,2-diphenyl-hexanenitrile

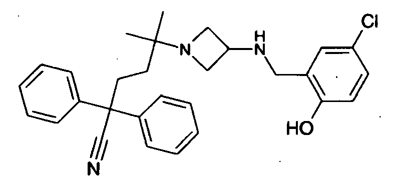

The title compound was prepared from the product of preparation 5 and 5-chloro-2-hydroxybenzaldehyde using a similar method to that described for example 3, as a colourless oil in 67% yield. ¹HNMR(400MHz, CDCl3) δ: 0.92(s, 6H), 1.27-1.37 (m, 2H), 2.40-2.52 (m, 2H), 2.82-2.97 (m, 2H), 3.25-3.40 (m, 3H), 3.84 (s, 2H), 6.73-6.78 (m, 1H), 6.95 (s, 1H), 7.07-7.15 (m, 1H), 7.25-7.44 (m, 10H); LRMS ESI m/z 474 [M+H]⁺

### Example 9

### 5-[3-(2-Hydroxy-benzylamino)-azetidin-1-yl]-5-methyl-2,2-diphenyl-hexanoic acid amide

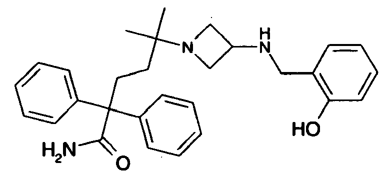

The title compound was prepared from the product of preparation 6 and 2-hydroxybenzaldehyde using a similar method to that described for example 3, as a colourless oil in 23% yield. ¹HNMR(400MHz, CDCl3) δ: 0.88 (s, 6H), 1.08-1.17 (m, 2H), 2.38-2.45 (m, 2H), 2.82-2.94 (m, 2H), 3.26-3.43 (m, 3H), 3.86 (s, 2H), 5.49-5.58 (br m, 2H), 6.74-6.83 (m, 2H), 6.92-6.97 (m, 1H), 7.13-7.18 (m, 1H), 7.23-7.37 (m, 10H); LRMS APCI m/z 458 [M+H]⁺

### Example 10

### 5-[3-(4-Fluoro-3-hydroxy-benzylamino)-azetidin-1-yl]-5-methyl-2,2-diphenyl-hexanoic acid amide

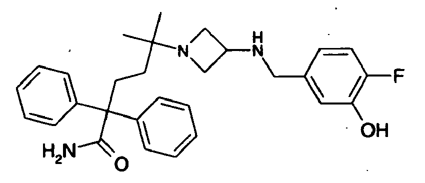

The title compound was prepared from the product of preparation 6 and 4-fluoro-3-hydroxybenzaldehyde (Bioorg. Med. Chem. 2001, 9, 677) using a similar method to that described for example 3, as a colourless oil in 11 % yield. ¹HNMR (400MHz, CDCl3) δ: 0.85 (s, 6H), 1.12-1.19 (m, 2H), 2.38-2.44 (m, 2H), 2.80-2.92 (m, 2H), 3.28-3.43 (m, 3H), 3.54 (s, 2H), 5.56-5.60 (br m, 2H), 6.60-6.67 (m, 1H), 6.80-6.87 (m, 1H), 6.88-6.97 (m, 1H), 7.18-7.37 (m, 10H); LRMS ESI m/z 476 [M+H]⁺

### Example 11

### 5-[3-(4-Chloro-3-methoxy-benzylamino)-azetidin-1-yl]-5-methyl-2,2-diphenyl-hexanoic acid amide

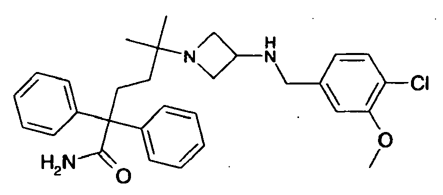

The title compound was prepared from the product of preparation 6 and 4-chloro-3-methoxybenzaldehyde using a similar method to that described for example 3, as a colourless oil in 54% yield. ¹HNMR(400MHz, CDCl3) δ: 0.84 (s, 6H), 1.07-1.18 (m, 2H), 2.38-2.45 (m, 2H), 2.75-2.83 (m, 2H), 3.25-3.33 (m, 2H), 3.34-3.40 (m, 1H), 3.64 (s, 2H), 3.87 (s, 3H), 5.45-5.60 (br m, 2H), 6.78-6.82 (m, 1H), 6.92 (s, 1H), 7.08-7.38 (m, 11H); LRMS ESI m/z 506 [M+H]⁺

### Example 12

### 5-[3-(4-Chloro-3-hydroxy-benzylamino)-azetidin-1-yl]-5-methyl-2,2-diphenyl-hexanoic acid amide

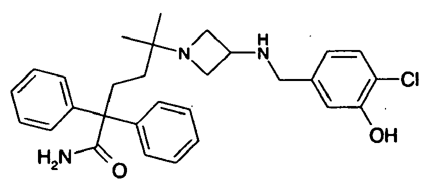

The product of example 11 (300mg, 0.59mmol) was dissolved in dichloromethane (10mL) at 0°C and boron tribromide (2.37mL, 2.37mmol, 1M in dichloromethane) was added. The reaction mixture was left to warm to 15°C over 2 hours. The reaction was quenched with water (5mL) and 0.88 ammonia (15mL) and the resulting solution was stirred at room temperature for 18 hours. The organic layer was separated, dried over magnesium sulphate and concentrated in vacuo. Purification of the residue by column chromatography on silica gel, eluting with dichloromethane:methanol:0.88 ammonia 100:0:0 to 80:20:2 followed by reverse phase HPLC, eluting with 0.05% diethylamine in acetonitrile:0.05% aqueous diethylamine 5:95 to 100:0 yielded the title compound as a colourless solid in 11% yield, 33mg.
1HNMR(400MHz, CDCl3) δ: 0.85 (s, 6H), 1.11-1.18 (m, 2H), 2.39-2.46 (m, 2H), 2.77-2.83 (m, 2H), 3.27-3.40 (m, 3H), 3.62 (s, 2H), 5.40-5.60 (br m, 2H), 6.75-6.79 (m, 1H), 6.95 (s, 1H), 7.20-7.37 (m, 11H); LRMS APCI m/z 492 [M+H]⁺

### Example 13

### 5-[3-(3-Methoxy-phenylsulfanyl)-azetidin-1-yl]-5-methyl-2,2-diphenyl-hexanenitrile

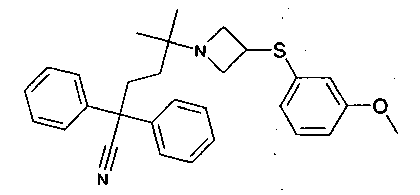

The product of preparation 3 (300mg, 0.73 mmol) was dissolved in N,N-dimethylformamide (5mL) and 3-methoxythiophenol (98µL, 0.80 mmol) and cesium carbonate (473mg, 1.5 mmol) were added. The reaction mixture was stirred at 80°C for 2 hours then concentrated in vacuo. The residue was diluted with water (20mL) and extracted with diethyl ether (3x30mL). The combined organic solution was dried over magnesium sulphate and concentrated in vacuo. Purification of the residue by column chromatography on silica gel, eluting with pentane:ethylacetate/methanol/0.88 ammonia (90/10/1) 100:0 to 1:5, afforded the title compound as a colourless solid in 66% yield, 220mg.
¹HNMR(400MHz, CDCl3) δ: 0.88 (s, 6H), 1.26-1.38 (m, 2H), 2.40-2.52 (m, 2H), 3.05-3.15 (m, 2H), 3.44-3.56 (m, 2H), 3.79 (s, 3H), 3.82-3.88 (m, 1H), 6.67-6.80 (m, 3H), 7.15-7.20 (m, 1H), 7.22-7.43 (m, 10H); LRMS ESI m/z 457 [M+H]⁺

### Example 14

### 5-[3-(3-Methoxy-phenylsulfanyl)-azetidin-1-yl]-5-methyl-2,2-diphenyl-hexanoic acid amide

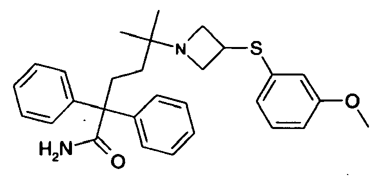

The product of example 13 (220mg, 0.482 mmol) was dissolved in 3-methyl-3-pentanol (5mL) and powdered potassium hydroxide added (535mg, 9.55mmol). The reaction mixture was stirred at 120°C for 18 hours then concentrated in vacuo. The residue was diluted with water (20mL) and extracted with ethyl acetate (3x30mL). The combined organics were dried over magnesium sulphate and concentrated in vacuo, affording the title compound as a colourless oil in 96% yield, 220mg.
¹HNMR(400MHz, CDCl₃) δ: 0.86 (s, 6H), 1.08-1.17 (m, 2H), 2.38-2.45 (m, 2H), 3.06-3.13 (m, 2H), 3.46-3.54 (m, 2H), 3.77 (s, 3H), 3.80-3.91 (m, 1H), 5.48-5.77 (br m, 2H), 6.67-6.78 (m, 3H), 7.13-7.19 (m, 1H), 7.20-7.38 (m, 10H); LRMS ESI m/z 475 [M+H]⁺

### Example 15

### 5-[3-(3-Hydroxy-phenylsulfanyl)-azetidin-1-yl]-5-methyl-2,2-diphenyl-hexanoic acid amide

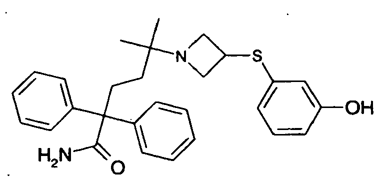

The product of example 14 (220mg, 0.46mmol) was dissolved in dichloromethane (3mL) at 0°C and boron tribromide (1.85mL, 1.85mmol, 1M in dichloromethane) was added. The reaction mixture was left to warm to 5°C over 2 hours. After cooling to -10°C, further boron tribromide (0.90mL, 0.90mmol) was added and reaction mixture was left to warm gradually to 5°C over 45 minutes. The reaction was treated with thiophenol (47µL, 0.46mmol) then 0.88 ammonia (20mL) and dichloromethane (5mL) and the resulting solution was stirred at room temperature for 18 hours. The organic layer was separated and the aqueous layer was extracted with dichloromethane (2x30mL). The combined organics were dried over magnesium sulphate and concentrated in vacuo. Purification of the residue by column chromatography on silica gel, eluting with pentane:ethylacetate/methano/0.88 ammonia (90/10/1) 100:0 to 40:60 yielded the title compound as a colourless foam in 92% yield, 196mg.
¹HNMR(400MHz, CDCl3) δ: 0.87 (s, 6H), 1.08-1.20 (m, 2H), 2.37-2.45 (m, 2H), 3.12-3.20 (m, 2H), 3.53-3.60 (m, 2H), 3.78-3.86 (m, 1H), 5.55-5.75 (br m, 1H), 7.67-5.95 (br m, 1H), 6.60-6.87 (m, 3H), 7.03-7.12 (m, 1H), 7.18-7.35 (m, 10H); LRMS ESI m/z 461 [M+H^{]+}

### Example 16

### 5-[3-(3-Chloro-4-hydroxy-benzylamino)-azetidin-1-yl]-5-methyl-2,2-diphenyl-hexanoic acid amide

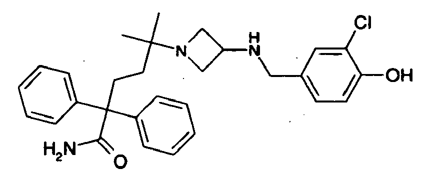

The title compound was prepared from the product of preparation 6 and 3-chloro-4-hydroxybenzaldehyde using a similar method to that described for example 3, as a colourless oil in 23% yield. ¹HNMR(400MHz, CDCl3) δ: 0.92 (s, 6H), 1.13-1.20 (m, 2H), 2.38-2.45 (m, 2H), 2.80-2.93 (m, 2H), 3.30-3.40 (m, 3H), 3.58 (s, 2H), 5.53-5.60 (br m, 2H), 6.83-6.85 (m, 1H), 6.99-7.03 (m, 1H), 7.20-7.38 (m, 11H); LRMS APCI m/z 492 [M+H]⁺

### Example 17

### 5-[3-(4-hydroxy-benzylamino)-azetidin-1-yl]-5-methyl-2,2-diphenyl-hexanoic acid amide

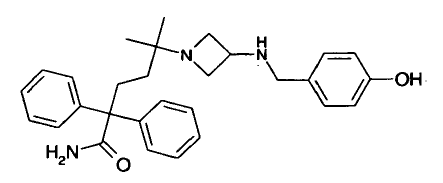

The title compound was prepared from the product of preparation 6 and 4-hydroxybenzaldehyde using a similar method to that described for example 3, as a colourless oil in 21% yield. ¹HNMR(400MHz, CDCl3) δ: 0.92 (s, 6H), 1.13-1.20 (m, 2H), 2.38-2.45 (m, 2H), 2.82-2.95 (m, 2H), 3.33-3.42 (m, 3H), 3.60 (s, 2H), 5.56-5.63 (br m, 2H), 6.64-6.68 (m, 2H), 7.01-7.05 (m, 2H), 7.20-7.38 (m, 10H); LRMS APCI m/z 458 [M+H]⁺

### Potency assay

M₃ potency was determined in CHO-K1 cells transfected with the NFAT-Betalactamase gene. CHO (Chinese Hamster Ovary) cells recombinantly expressing the human muscarinic M₃ receptor were transfected with the NFAT_β-Lac_Zeo plasmid. Cells were grown in DMEM with Glutamax-1, supplemented with 25mM HEPES(Life Technologies 32430-027), containing 10% FCS (Foetal Calf Serum; Sigma F-7524), 1nM Sodium pyruvate (stigmas-8636), NEAA (non-Essential Amino Acids; Invitrogen 11140-035) and 200µg/ml Zeocin (Invitrogen R250-01).

### hM₃ β-Lac Assay Protocol

Cells were harvested for assay when they reached 80-90% confluency using enzyme free cell Dissociation Solution (Life technologies 13151-014) incubated with the cells for 5 min at 37°C in an atmosphere containing 5% CO₂. Detached cells were collected in warmed growth media and centrifuged at 2000rpm for 10min, washed in PBS (Phosphate Buffered Saline; Life Technologies 14190-094) and centrifuged again as just described. The cells were resuspended at 2x10⁵ cells/ml in growth medium (composition as described above). 20µl of this cell suspension was added to each well of a 384 well black clear bottomed plate (Greiner Bio One 781091-PFI). The assay buffer used was PBS supplemented with 0.05% Pluronic F-127 (Sigma 9003-11-6) and 2.5% DMSO. Muscarinic M₃ receptor signalling was stimulated using 80nM carbamyl choline (Aldrich N240-9) incubated with the cells for 4h at 37°C /5% CO₂ and monitored at the end of the incubation period using a Tecan SpectraFluor+ plate reader (λ - excitation 405nm, emission 450nm and 503nm). M₃ receptor antagonists under test were added to the assay at the beginning of the 4h incubation period and compound activity measured as the concentration dependent inhibition of the carbamyl choline induced signal. Inhibition curves were plotted and IC₅₀ values generated using a 4-parameter sigmoid fit and converted to Ki values using the Cheng-Prusoff correction and the K_{D} value for carbamyl choline in the assay.

It has thus been found that carboxamide derivatives of formula (I) according to the present invention that have been tested in the above assay show M₃ receptor antagonist activity as listed in the table below:

| Example Number | Cell based β-lactamase M₃ Ki (nM) |
|---|---|
| 2 | 0.556 |
| 3 | 2.08 |
| 4 | 56.8 |
| 5 | 2.53 |
| 6 | 0.727 |
| 7 | 4.37 |
| 8 | >315 |
| 9 | 0.594 |
| 10 | 0.396 |
| 12 | 0.905 |
| 15 | 0.368 |

### Guinea Pig Trachea assay

Male, Dunkin-Hartley guinea-pigs weighing 350-450g are culled in a rising concentration of CO₂, followed by exsanguinations of the vena cava. Tracheas are dissected from the larynx to the entry point into the chest cavity and then placed in fresh, oxygenated, modified Krebs buffer solution (Krebs containing 10µM propranolol, 10µM guanethidine and 3µM indomethacin) at room temperature. The tracheas are opened by cutting through the cartilage opposite the trachealis muscle. Strips approximately 3-5 cartilage rings wide are cut. A cotton thread is attached to the cartilage at one end of the strip for attachment to the force transducer and a cotton loop made at the other end to anchor the tissue in the organ bath. The strips are mounted in 5ml organ baths filled with warm (37°C) aerated modified Krebs. The pump flow rate is set to 1.0 ml/ min and the tissues washed continuously. Tissues are placed under an initial tension of 1000mg. Tissues are re-tensioned after 15 and 30 minutes, then allowed to equilibrate for a further 30-45 minutes.
Tissues are subjected to electrical field stimulation (EFS) of the following parameters: 10s trains every 2 minutes, 0.1ms pulse width, 10Hz and 10-30V. The voltage is raised 5V every 10min within the stated range until a maximum contractile response for each tissue is observed. This just maximum voltage for each tissue is then used throughout the remainder of the experiment. Following equilibration to EFS for 20min, the pump is stopped, and after 15min control readings are taken over a 8-10 min period (4-5 responses). Compound is then added to each tissue as a bolus dose at 30xKi (determined at the human M₃ receptor expressed in CHO cells in a filtration binding assay), and left to incubate for 2h. Compound is then washed from tissues using a rapid wash with modified Krebs for 1min and flow is restored to 1ml/min for the remainder of the experiment. At the end of the experiment tissues are challenged with histamine (1µM) to determine viability. Readings taken during the experiment are automatically collected using Notocord ® software. The raw data are converted into percent response taking into account measurements of inhibition of the EFS response. After starting washout, the times taken for the tissue to recover by 25% from the inhibition induced are recorded and used as a measure of compound duration of action. Tissue viability limits the duration of the experiment to 16h post-compound washout. Compounds are typically tested at n=2 to 5 to estimate duration of action.

### Alternatively the following Guinea Pig Trachea assay can also be used:

Trachea were removed from male Dunkin-Hartley guinea-pigs (wt 350-450g) and following removal of adherent connective tissue, an incision was made through the cartilage opposite the trachealis muscle and tracheal strips 3-5 cartilage rings wide prepared. The tracheal strips were suspended between an isometric strain gauge and a fixed tissue hook with the muscle in the horizontal plane in 5ml tissue baths under an initial tension of 1g and bathed in warmed (37°C) aerated (95%O₂/5%CO₂) Krebs solution containing 3µM indomethacin and 10µM guanethidine. The tissues were positioned between parallel platinum wire electrodes (∼1cm gap). A constant 1ml/min flow of fresh Krebs solution (of the above composition) was maintained through the tissue baths using peristaltic pumps. The tissues were allowed to equilibrate for an hour with re-tensioning to 1g at 15min and 30min from the start of the equilibration period. At the end of the equilibration, tissues were electrically field stimulated (EFS) using the following parameters: 10V, 10Hz 0.1ms pulse width with 10sec trains every 2 min. In each tissue a voltage response curve was constructed over the range 10v - 30V (keeping all other stimulation parameters constant) to determine a just maximal stimulation. Using these stimulation parameters EFS responses were 100% nerve mediated and 100% cholinergic as confirmed by blockade by 1µM tetrodotoxin or 1µM atropine. Tissues were then repeatedly stimulated at 2 min intervals until the responses were reproducible. The peristaltic pump was stopped 20 min prior to the addition of the study compound and the average twitch contraction over the last 10min recorded as the control response. The study compound was added to the tissue baths, with each tissue receiving a single concentration of compound and allowed to equilibrate for 2h. At 2h post addition the inhibition of the EFS response was recorded and IC₅₀ curves generated using a range of compound concentrations over tracheal strips from the same animal. The tissues were then rapidly washed and the 1ml/min perfusion with Krebs solution re-established. Tissues were stimulated for a further 16h and recovery of the EFS response recorded. At the end of the 16h, 10µM histamine was added to the baths to confirm tissue viability. The just max concentration (tested concentration giving a response > 70% inhibition but less than 100%) of antagonist was identified from the IC₅₀ curve and the time to 25% recovery of the induced inhibition (T₂₅) calculated in tissues receiving this concentration. Compounds are typically tested at n=2 to 5 to estimate duration of action.

## Claims

1. A compound of formula (I) 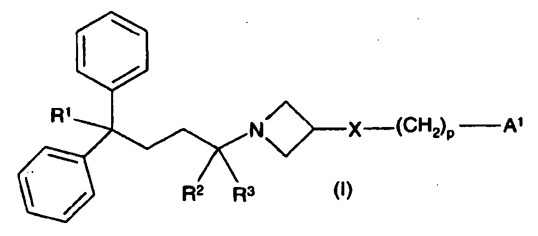 wherein,
- R¹ is CN or CONH₂;
- R² and R³ are methyl, or, R² and R³ may also together form with the carbon atom to which they are linked a cyclopentane ring;
- X is NH orS;
- p is 0 or 1;
- A¹ is selected from
a) phenyl optionally substituted with 1, 2 or 3 groups independently selected from halo, CN, CF₃, OR⁴, SR⁴, OCF₃, (C₁-C₄)alkyl and phenyl optionally substituted with OH;
b) naphthyl optionally substituted with 1 or 2 groups independently selected from halo, CN, CF₃, OR⁴, SR⁴, OCF₃ and (C₁-C₄)alkyl;
c) a 9 or 10-membered bicyclic aromatic heterocyclic group, containing from 1 to 3 heteroatoms independently selected from O, S or N, said heterocyclic group being optionally substituted with 1 or 2 substituents selected from OR⁴, (C₁-C₄)alkyl and halo;
- R⁴ is H or (C₁-C₄)alkyl;
or the pharmaceutically acceptable salts or solvates thereof.

2. A compound according to claim 1 or the pharmaceutically acceptable salts or solvates thereof where R¹ is CONH₂.

3. A compound according to claim 1 or 2 or the pharmaceutically acceptable salts or solvates thereof where p is 0 and X is S.

4. A compound according to claim 1 or 2 or the pharmaceutically acceptable salts or solvates thereof where p is 1 and X is NH.

5. A compound according to any one of claims 1 to 4 or the pharmaceutically acceptable salts or solvates thereof where A¹ is phenyl optionally substituted with 1 to 3 groups, independently selected from F, Cl, CF₃, OH, OCH₃, OCF₃ and CH₃.

6. A compound according to any one of claims 1 to 5 or the pharmaceutically acceptable salts or solvates thereof where A¹ is phenyl optionally substituted with 1 to 2 groups independently selected from F, Cl, CF₃; OH, OCH₃, OCF₃ and CH₃.

7. A compound according to any one of claims 1 to 6 or the pharmaceutically acceptable salts or solvates thereof where A¹ is phenyl optionally substituted with 1 to 2 groups independently selected from F, Cl and OH.

8. A compound according to any one of claims 1 to 7 or the pharmaceutically acceptable salts or solvates thereof where R² and R³ are methyl.

9. A compound according to claim 1 or the pharmaceutically acceptable salts or solvates thereof, said compound being selected from,
5-(3-Benzylamino-azetidin-1-yl)-5-methyl-2,2-diphenyl-hexanenitrile;
5-(3-Benzylamino-azetidin-1-yl)-5-methyl-2,2-diphenyl-hexanoic acid amide;
5-[3-(2-Chloro-3-hydroxy-banzylamino)-azetidin-1-yl]-5-methyl-2,2-diphenyl-hexanoic acid amide;
5-[3-(5-Chloro-2-hydroxy-benzylamino)-azetidin-1-yl]-5-methyl-2,2-diphenyl-hexanoic acid amide;
5-[3-(5-Fluoro-2-hydroxy-benzylamino)-azetidin-1-yl]-5-methyl-2,2-diphenyl-hexanoic acid amide;
5-[3-(3-Hydroxy-benzylamino)-azetidin-1-yl]-5-methyl-2,2-diphenyl-hexanoic acid amide;
5-[3-(5-Fluoro-2-hydroxy-benzylamino)-azetidin-1-yl]-5-methyl-2,2-diphenyl-hexanenitrile;
5-[3-(5-Chloro-2-hydroxy-benzylamino)-azetidin-1-yl]-5-methyl-2,2-diphenyl-hexanenitrile;
5-[3-(2-Hydroxy-benrylamino)-azetidin-1-yl]-5-methyl-2,2-diphenyl-hexanoic acid amide;
5-[3-(4-Fluoro-3-hydroxy-benzylamino)-azetidin-1-yl]-5-methyl-2,2-diphenyl-hexanoic acid amide;
5-[3-(4-Chloro-3-methoxy-benzylamino)-azatidin-1-yl)-5-methyl-2.2-diphenyl-hexanoic acid amide;
5-[3-(4-Chloro-3-hydroxy-benzylamino)-azetidin-1-yl]-5-methyl-2,2-diphenyl-hexanoic acid amide;
5-[3-(3-Methoxy-phenylsulfanyl)-azetidin-1-yl]-5-methyl-2,2-diphenyl-hexanenitrile;
5-[3-(3-Methoxy-phenylsufanyl)-azetidin-1-yl]-5-methyl-2,2-diphenyl-hexanoic acid amide, 5-[3-(3-Hydroxy-phenylsulfanyl)-azetidin-1-yl]-5-methyl-2,2-diphenyl-hexanoic acid amide,
5-[3-(3-Chloro-4-hydroxy-benzylamino)-azetidin-1-yl]-5-methyl-2,2-diphenyl-hexanoic acid amide, and,
5-[3-(4-hydroxy-benzylamino)-azetidin-1-yl]-5-methyl-2,2-diphenyl-hexanoic acid amide.

10. A pharmaceutical composition comprising at least an effective amount of a compound of the formula (I) as described in any one of claims 1 to 9 or the pharmaceutically acceptable salts or solvates thereof

11. A compound of the formula (I) as described in any one of claims 1 to 9 or the pharmaceutically acceptable salts or solvates thereof, for use as a medicament.

12. A compound of the formula (I) as described in any one of claims 1 to 9 or the pharmaceutically acceptable salts or solvates thereof, for use in the treatment of treatment of diseases, disorders, and conditions selected from the group consisting of :
- chronic or acute bronchoconstriction, chronic bronchitis, small airways obstruction, and emphysema,
- obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated or not associated with COPD, COPD that is **characterized by** irreversible, progressive airways obstruction, adult respiratory distress syndrome (ARDS), exacerbation of airways hyper-reactivity consequent to other drug therapy and airways disease that is associated with pulmonary hypertension,
- bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, infectious asthmatic bronchitis, productive bronchitis, staphylococcus or streptococcal bronchitis and vesicular bronchitis,
- asthma of whatever type, etiology, or pathogenesis, in particular asthma that is a member selected from the group consisting of atopic asthma, non-atopic asthma, allergic asthma, atopic bronchial lgE-mediated asthma, bronchial asthma, essential asthma, true asthma, intrinsic asthma caused by pathophysiologic disturbances, extrinsic asthma caused by environmental factors, essential asthma of unknown or inapparent cause, non-atopic asthma, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma, wheezy infant syndrome and bronchiolytis,
- acute lung injury,
- bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis.

13. Combination of a compound according to any one of claims 1 to 9 or the pharmaceutically acceptable salts or solvates thereof, with other therapeutic agent(s) selected from:
(a) 5-Lipoxygenase (5-LO) inhibitors or 5-lipoxygenase activating protein (FLAP) antagonists,
(b) Leukotriene antagonists (LTRAs) including antagonists of LTB₄, LTC₄, LTD₄, and LTE₄,
(c) Histamine receptor antagonists including H1 and H3 antagonists,
(d) α₁- and α₂-adrenoceptor agonist vasoconstrictor sympathomimetic agents for decongestant use,
(e) short or long acting β₂ agonists,
(f) PDE inhibitors, e.g. PDE3, PDE4 and PDE5 inhibitors,
(g) Theophylline,
(h) Sodium cromoglycate,
(i) COX inhibitors both non-selective and selective COX-1 or COX-2 inhibitors (NSAIDs),
(j) Oral and inhaled glucocorticosteroids,
(k) Monoclonal antibodies active against endogenous inflammatory entities,
(l) Anti-tumor necrosis factor (anti-TNF-α) agents,
(m) Adhesion molecule inhibitors including VLA-4 antagonists,
(n) Kinin-B₁-and B₂ -receptor antagonists,
(o) Immunosuppressive agents,
(p) Inhibitors of matrix metalloproteases (MMPs),
(q) Tachykinin NK₁, NK₂ and NK₃ receptor antagonists,
(r) Elastase inhibitors,
(s) Adenosine A2a receptor agonists,
(t) Inhibitors of urokinase,
(u) Compounds that act on dopamine receptors, e.g. D2 agonists,
(v) Modulators of the NFκB pathway, e.g. IKK inhibitors,
(w) modulators of cytokine signalling pathyways such as p38 MAP kinase, syk kinase, or JAK kinase inhibitors,
(x) Agents that can be classed as mucolytics or anti-tussive,
(y) Antibiotics,
(z) Prostaglandin antagonists such as DP1, DP2 or CRTH2 antagonists,
(aa) HDAC inhibitors,
(bb)PI3 kinase inhibitors, and,
(cc) CXCR2 antagonists.

14. An intermediate of formula 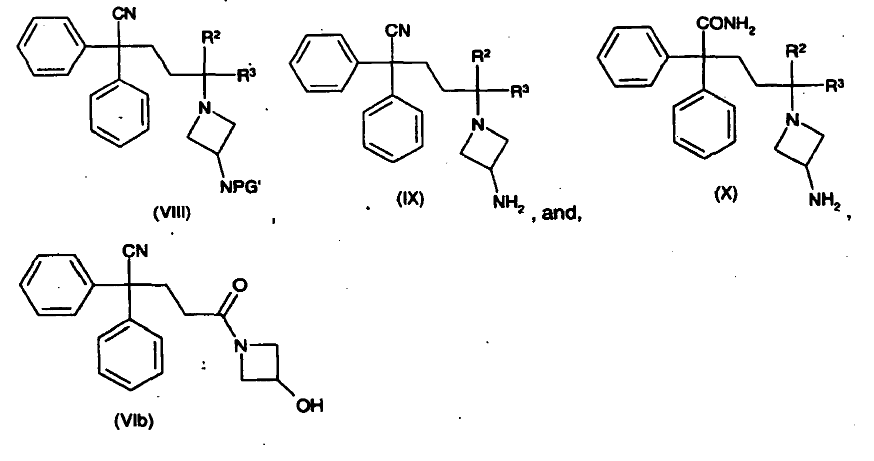 wherein R² and R³ are as defined for compounds of formula (I) and PG' is a suitable amine protecting group.

## Patentansprüche

1. Verbindung der Formel (I) 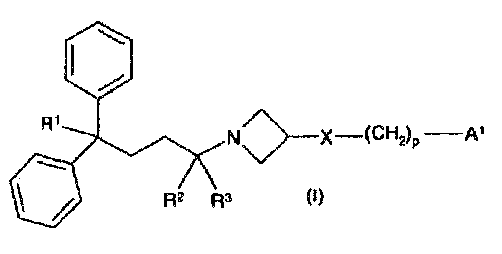 worin
- R¹ CN oder CONH₂ ist;
- R² und R³ Methyl sind oder R² und R³ auch zusammen mit dem Kohlenstoffatom, an das sie gebunden sind, einen Cyclopentanring bilden können;
- X NH oder S ist;
- p 0 oder 1 ist;
- A¹ausgewählt ist aus
a) Phenyl, gegebenenfalls substituiert mit 1, 2 oder 3 Gruppen, unabhängig ausgewählt aus Halogen, CN, CF₃, OR⁴, SR⁴ OCF₃ , (C₁-C₄) -Alkyl und Phenyl, gegebenenfalls substituiert mit OH;
b) Naphthyl, gegebenenfalls substituiert mit 1 oder 2 Gruppen, unabhängig ausgewählt aus Halogen, CN, CF₃, OR⁴, SR⁴, OCF₃ und (C₁-C₄) -Alkyl ;
c) einer 9- oder 10-gliedrigen bicyclischen aromatischen heterocyclischen Gruppe, die 1 bis 3 Heteroatome, unabhängig ausgewählt aus 0, S und N, enthält, wobei die heterocyclische Gruppe gegebenenfalls mit 1 oder 2 Substituenten, ausgewählt aus OR⁴, (C₁-C₄)-Alkyl und Halogen, substituiert ist;
- R⁴ H oder (C₁-C₄) -Alkyl ist,
oder die pharmazeutisch verträglichen Salze oder Solvate davon.

2. Verbindung gemäß Anspruch 1 oder die pharmazeutisch verträglichen Salze oder Solvate davon, wobei R¹ CONH₂ ist.

3. Verbindung gemäß Anspruch 1 oder 2 oder die pharmazeutisch verträglichen Salze oder Solvate davon, wobei p 0 ist und X S ist.

4. Verbindung gemäß Anspruch 1 oder 2 oder die pharmazeutisch verträglichen Salze oder Solvate davon, wobei p 1 ist und X NH ist.

5. Verbindung gemäß einem der Ansprüche 1 bis 4 oder die pharmazeutisch verträglichen Salze oder Solvate davon, wobei A¹ Phenyl ist, das gegebenenfalls mit 1 bis 3 Gruppen substituiert ist, die unabhängig ausgewählt sind aus F, Cl, CF₃, OH, OCH₃ , OCF₃ und CH₃ .

6. Verbindung gemäß einem der Ansprüche 1 bis 5 oder die pharmazeutisch verträglichen Salze oder Solvate davon, wobei A¹ Phenyl ist, das gegebenenfalls mit 1 bis 2 Gruppen, unabhängig ausgewählt aus F, Cl, CF₃, OH, OCH₃, OCF₃ und CH₃, substituiert ist.

7. Verbindung gemäß einem der Ansprüche 1 bis 6 oder die pharmazeutisch verträglichen Salze oder Solvate davon, wobei A¹ Phenyl ist, das gegebenenfalls mit 1 bis 2 Gruppen, unabhängig ausgewählt aus F, Cl und OH, substituiert ist.

8. Verbindung gemäß einem der Ansprüche 1 bis 7 oder die pharmazeutisch verträglichen Salze oder Solvate davon, wobei R² und R³ Methyl sind.

9. Verbindung gemäß Anspruch 1 oder die pharmazeutisch verträglichen Salze oder Solvate davon, wobei die Verbindung ausgewählt ist aus
5-(3-Benzylamino-azetidin-1-yl)-5-methyl-2,2-diphenylhexannitril;
5-(3-Benzylamino-azetidin-1-yl)-5-methyl-2,2-diphenylhexansäureamid;
5-[3-(2-Chlor-3-hydroxy-benzylamino)-azetidin-1-yl]-5-methyl-2,2-diphenylhexansäureamid;
5-[3-(5-Chlor-2-hydroxy-benzylamino)-azetidin-1-yl]-5-methyl-2,2-diphenylhexansäureamid;
5-[3-(5-Fluor-2-hydroxy-benzylamino)-azetidin-1-yl]-5-methyl-2,2-diphenyl-hexansäureamid;
5-[3-(3-Hydroxy-benzylamino)-azetidin-1-yl]-5-methyl-2,2-diphenyl-hexansäureamid;
5-[3-[5-Fluor-2-hydroxy-benzylamino)-azetidin-1-yl]-5-methyl-2,2-diphenyl-hexannitril;
5-[3-(5-Chlor-2-hydroxy-benzylamino)-azetidin-l-yl]-5-methyl-2,2-diphenyl-hexannitril;
5-[3-(2-Hydroxy-benzylamino)-azetidin-1-yl]-5-methyl-2,2-diphenyl-hexansäureamid;
5-[3-(4-Fluor-3-hydroxy-benzylamino)-azetidin-1-yl]-5-methyl-2,2-diphenyl-hexansäureamid;
5-[3-(4-Chlor-3-methoxy-benzylamino)-azetidin-1-yl]-5-methyl-2,2-diphenyl-hexansäureamid;
5-[3-(4-Chlor-3-hydroxy-benzylamino)-azetidin-1-yl]-5-methyl-2,2-diphenyl-hexansäureamid;
5-[3-(3-Methoxy-phenylsulfanyl)-azetidin-1-yl]-5-methyl-2,2-diphenyl-hexannitril;
5-[3-(3-Methoxy-phenylsulfanyl)-azetidin-1-yl]-5-methyl-2,2-diphenyl-hexansäureamid;
5-[3-(3-Hydroxy-phenylsulfanyl)-azetidin-1-yl]-5-methyl-2,2-diphenylhexansäureamid;
5-[3-(3-Chlor-4-hydroxy-benzylamino)-azetidin-1-yl]-5-methyl-2,2-diphenyl-hexansäureamid und
5-[3-(4-Hydroxy-benzylamino)-azetidin-1-y1]-5-methyl-2,2-diphenyl-hexansäureamid.

10. Pharmazeutische Zusammensetzung, umfassend wenigstens eine wirksame Menge einer Verbindung der Formel (I), wie sie in einem der Ansprüche 1 bis 9 beschrieben ist, oder der pharmazeutisch verträglichen Salze oder Solvate davon.

11. Verbindung der Formel (I), wie sie in einem der Ansprüche 1 bis 9 beschrieben ist, oder die pharmazeutisch verträglichen Salze oder Solvate davon zur Verwendung als Medikament.

12. Verbindung der Formel (I), wie sie in einem der Ansprüche 1 bis 9 beschrieben ist, oder die pharmazeutisch verträglichen Salze oder Solvate davon zur Verwendung bei der Behandlung von Erkrankungen, Störungen und Zuständen, ausgewählt aus der Gruppe, bestehend aus:
- chronischer oder akuter Bronchokonstriktion, chronischer Bronchitis, Obstruktion der kleinen Luftwege und Emphysem,
- obstruktiven oder inflammatorischen Luftwegserkrankungen jedweden Typs, jedweder Ätiologie oder jedweder Pathogenese, insbesondere eine obstruktive oder inflammatorische Luftwegserkrankung, die ein Mitglied ist, ausgewählt aus der Gruppe, bestehend aus chronischer eosinophiler Pneumonie, chronischobstruktiver Lungenerkrankung (COPD), COPD, die chronische Bronchitis, Lungenemphysem oder Dyspnoe, assoziiert oder nicht assoziiert mit COPD umfasst, COPD, die durch irreversible, progressive Luftwegsobstruktion charakterisiert ist, Atemnotsyndrom bei Erwachsenen (ARDS), Verschlimmerung der Atemwegshyperreaktivität als Folge einer anderen Arzneimitteltherapie und Luftwegserkrankung, die mit Lungenhochdruck assoziiert ist,
- Bronchitis jedweden Typs, jedweder Ätiologie oder jedweder Pathogenese, insbesondere Bronchitis, die ein Mitglied ist, ausgewählt aus der Gruppe, bestehend aus akuter Bronchitis, akuter Laryngotrachealbronchitis, Erdnussbronchitis, Bronchitis catarrhalis, kruppöser Bronchitis, trockener Bronchitis, infektiöser asthamatischer Bronchitis, produktiver Bronchitis, Staphylokokken- oder Streptokokkenbronchitis und vesikulärer Bronchitis,
- Asthma jedweden Typs, jedweder Ätiologie oder jedweder Pathogenese, insbesondere Asthma, das ein Mitglied ist, ausgewählt aus der Gruppe, bestehend aus atopischem Asthma, nicht-atopischem Asthma, allergischem Asthma, atopischem bronchialem IgE-vermittelten Asthma, Bronchialasthma, essentiellem Asthma, echtem Asthma, intrinsischem Asthma, verursacht durch pathophysiologische Störungen, extrinsischem Asthma, verursacht durch Umweltfaktoren, essentiellem Asthma unbekannter oder inapparenter Ursache, nicht-atopischem Asthma, Bronchitisasthma, emphysematösem Asthma, anstrengungsinduziertem Asthma, durch Allergen induziertem Asthma, durch kalte Luft induziertem Asthma, Berufsasthma, infektiösem Asthma, verursacht durch bakterielle, Pilz-, Protozon- oder Virusinfektion, nicht-allergischem Asthma, beginnendem Asthma, Wheezy-Infant-Syndrom und Bronchiolytis,
- akuter Lungenverletzung,
- Bronchiektasie jedweden Typs, jedweder Ätiologie oder jedweder Pathogenese, insbesondere Bronchiektasie, die ein Mitglied ist, ausgewählt aus der Gruppe, bestehend aus zylindrischer Bronchiektasie, sackförmiger Bronchiektasie, spindelförmiger Bronchiektasie, kapillarförmiger Bronchiektasie, cystischer Bronchiektasie, trockener Bronchiektasie und follikulärer Bronchiektasie.

13. Kombination einer Verbindung gemäß einem der Ansprüche 1 bis 9 oder der pharmazeutisch verträglichen Salze oder Solvate davon mit einem anderen therapeutischen Mittel (mit anderen therapeutischen Mitteln), ausgewählt aus:
(a) 5-Lipoxygenase (5-LO)-Inhibitoren oder 5-Lipoxygenaseaktivierenden Protein (FLAP)-Antagonisten,
(b) Leukotrienantagonisten (LTRAs), einschließlich Antagonisten von LTB₄, LTC₄ , LTD₄ und LTE₄ ,
(c) Histaminrezeptorantagonisten, einschließlich H1- und H3-Antagonisten,
(d) α₁-und α₂-Adrenoceptoragonist-Vasokonstriktor-Sympathomimetika zur Verwendung als Dekongestionsmittel,
(e) kurz oder lang wirkenden β₂-agonisten,
(f) PDE-Inhibitoren, zum Beispiel PDE3-, PDE4- und PDE5-Inhibitoren,
(g) Theophyllin,
(h) Natriumcromoglycat,
(i) COX-Inhibitoren, sowohl nicht-selektive als auch selektive COX-1- oder COX-2-Inhibitoren (NSAR),
(j) oralen und inhalierten Glucocorticosteroiden,
(k) monoklonalen Antikörpern, die gegen endogene inflammatorische Einheiten aktiv sind,
(l) Anti-Tumornekrosefaktor (Anti-TNF-α-Mitteln,
(m) Adhäsionsmolekülinhibitoren, einschließlich VLA-4-Antagonisten,
(n) Kinin-B₁- und -B₂-Rezeptorantagonisten,
(o) Immunsuppressiva,
(p) Inhibitoren von Matrixmetalloproteasen (MMPs),
(q) Tachykinin-NK₁-, -NK₂- und -NK₃-Rezeptorantagonisten,
(r) Elastaseinhibitoren,
(s) Adenosin-A2a-Rezeptoragonisten,
(t) Inhibitoren von Urokinase,
(u) Verbindungen, die auf Dopaminrezeptoren wirken, zum Beispiel D2-Agonisten,
(v) Modulatoren des NFκB-Wegs, zum Beispiel IKK-Inhibitoren,
(w) Modulatoren der Cytokinsignalübertragungswege, zum Beispiel p38-MAP-Kinase-, syk-Kinase- oder JAK-Kinaseinhibitoren,
(x) Mitteln, die als Mucolytika oder Antitussiva klassifiziert werden können,
(y) Antibiotika,
(z) Prostaglandinantagonisten, zum Beispiel DP1-, DP2- oder CRTH2-Antagonisten,
(aa) HDAC-Inhibitoren,
(bb) P13-Kinaseinhibitoren und
(cc) CXCR2-Antagonisten.

14. Intermediat der Formel 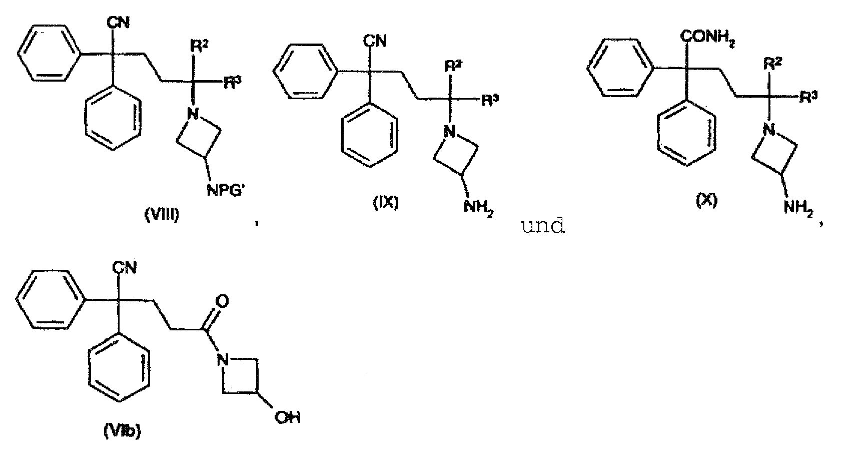 worin R² und R³ wie für Verbindungen der Formel (I) definiert sind und PG' eine geeignete Aminschutzgruppe ist.

## Revendications

1. Composé de formule (I) 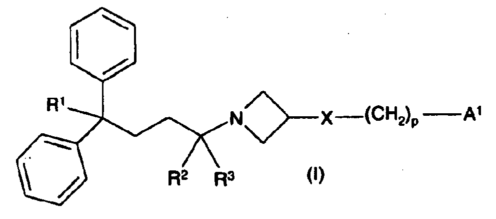 dans laquelle
- R¹ représente un groupe CN ou CONH₂ ;
- R² et R³ représentent des groupes méthyle, ou bien R² et R³ peuvent également former, conjointement avec l'atome de carbone auquel ils sont liés, un noyau cyclopentane ;
- X représente NH ou -S ;
- p est égal à 0 ou 1 ;
- A¹ est choisi entre
a) un groupe phényle facultativement substitué avec 1, 2 ou 3 groupes choisis indépendamment entre des groupes halogéno, CN, CF₃, OR⁴, SR⁴, OCF₃, alkyle en C₁ à C₄ et phényle facultativement substitué avec un substituant OH ;
b) un groupe naphtyle facultativement substitué avec 1 ou 2 groupes choisis indépendamment entre des groupes halogéno, CN, CF₃, OR⁴, SR⁴, OCF₃ et alkyle en C₁ à C₄ ;
c) un groupe hétérocyclique aromatique bicyclique nona- ou décagonal, contenant 1 à 3 hétéroatomes choisis indépendamment entre O, S et N, ledit groupe hétérocyclique étant facultativement substitué avec 1 ou 2 substituants choisis entre des substituants OR⁴, alkyle en C₁ à C₄ et halogéno ;
- R⁴ représente H un groupe alkyle en C₁ à C₄ ;
ou ses sels ou produits de solvatation pharmaceutiquement acceptables.

2. Composé suivant la revendication 1 ou ses sels ou produits de solvatation pharmaceutiquement acceptables, dans lesquels R¹ représente un groupe CONH₂.

3. Composé suivant la revendication 1 ou 2 ou ses sels ou produits de solvatation pharmaceutiquement acceptables, dans lesquels p est égal à 0 et X représente S.

4. Composé suivant la revendication 1 ou 2 ou ses sels ou produits de solvatation pharmaceutiquement acceptables, dans lesquels p est égal à 1 et X représente un groupe NH.

5. Composé suivant l'une quelconque des revendications 1 à 4 ou ses sels ou produits de solvatation pharmaceutiquement acceptables, dans lesquels A¹représente un groupe phényle facultativement substitué avec 1 à 3 groupes choisis indépendamment entre des groupes F, Cl, CF₃, OH, OCH₃, OCF₃ et CH₃.

6. Composé suivant l'une quelconque des revendications 1 à 5 ou ses sels ou produits de solvatation pharmaceutiquement acceptables, dans lesquels A¹ représente un groupe phényle facultativement substitué avec 1 ou 2 groupes choisis indépendamment entre des groupes F, Cl, CF₃, OH, OCH₃ et CH₃ .

7. Composé suivant l'une quelconque des revendications 1 à 6 ou ses sels ou produits de solvatation pharmaceutiquement acceptables, dans lesquels A¹ représente un groupe phényle facultativement substitué avec 1 ou 2 groupes choisis indépendamment entre des groupes F, Cl et OH.

8. Composé suivant l'une quelconque des revendications 1 à 7 ou ses sels ou produits de solvatation pharmaceutiquement acceptables, dans lesquels R² et R³ représentent des groupes méthyle.

9. Composé suivant la revendication 1 ou ses sels ou produits de solvatation pharmaceutiquement acceptables, ledit composé étant choisi entre :
le 5-(3-benzylamino-azétidine-1-yl)-5-méthyl-2,2-diphényl-hexanenitrile ;
l'amide d'acide 5-(3-benzylamino-azétidine-1-yl)-5-méthyl-2,2-diphényl-hexanoïque ;
l'amide d'acide 5-[3-(2-chloro-3-hydroxy-benzylamino)-azétidine-1-yl]-5-méthyl-2,2-diphényl-hexanoïque ;
l'amide d'acide 5-[3-(5-chloro-2-hydroxy-benzylamino)-azétidine-1-yl]-5-méthyl-2,2-diphényl-hexanoïque ;
l'amide d'acide 5-[3-(5-fluoro-2-hydroxy-benzylamino)-azétidine-1-yl]-5-méthyl-2,2-diphényl-hexanoïque ;
l'amide d'acide 5-[3-(3-hydroxy-benzylamino)-azétidine-1-yl]-5-méthyl-2,2-diphényl-hexanoïque ;
le 5-[3-(5-fluoro-2-hydroxy-benzylamino)-azétidine-1-yl]-5-méthyl-2,2-diphényl-hexanenitrile ;
le 5-[3-(5-chloro-2-hydroxy-benzylamino)-azétidine-1-yl]-5-méthyl-2,2-diphényl-hexanenitrile ;
l'amide d'acide 5-[3-(2-hydroxy-benzylamino)-azétidine-1-yl]-5-méthyl-2,2-diphényl-hexanoïque ;
l'amide d'acide 5-[3-(4-fluoro-3-hydroxy-benzylamino)-azétidine-1-yl]-5-méthyl-2,2-diphényl-hexanoïque ;
l'amide d'acide 5-[3-(4-chloro-3-méthoxy-benzylamino)-azétidine-1-yl]-5-méthyl-2,2-diphényl-hexanoïque ;
l'amide d'acide 5-[3-(4-chloro-3-hydroxy-benzylamino)-azétidine-1-yl]-5-méthyl-2,2-diphényl-hexanoïque ;
le 5-[3-(3-méthoxy-phénylsulfanyl)-azétidine-1-yl]-5-méthyl-2,2-diphényl-hexanenitrile ;
l'amide d'acide 5-[3-(3-méthoxy-phénylsulfanyl)-azétidine-1-yl]-5-méthyl-2,2-diphényl-hexanoïque ;
l'amide d'acide 5-[3-(3-hydroxy-phénylsulfanyl)-azétidine-1-yl]-5-méthyl-2,2-diphényl-hexanoïque ;
l'amide d'acide 5-[3-(3-chloro-4-hydroxy-benzylamino)-azétidine-1-yl]-5-méthyl-2,2-diphényl-hexanoïque ; et
l'amide d'acide 5-[3-(4-hydroxy-benzylamino)-azétidine-1-yl]-5-méthyl-2,2-diphényl-hexanoïque.

10. Composition pharmaceutique comprenant au moins une quantité efficace d'un composé de formule (I) répondant à la description figurant dans l'une quelconque des revendications 1 à 9 ou d'un de ses sels ou produits de solvatation pharmaceutiquement acceptables.

11. Composé de formule (I) répondant à la description figurant dans l'une quelconque des revendications 1 à 9 ou ses sels ou produits de solvatation pharmaceutiquement acceptables, destiné à être utilisé comme médicament.

12. Composé de formule (I) répondant à la description figurant dans l'une quelconque des revendications 1 à 9 ou ses sels ou produits de solvatation pharmaceutiquement acceptables, destiné à être utilisé dans le traitement de maladies, de troubles, et d'affections choisis dans le groupe consistant en :
- la bronchoconstriction chronique ou aiguë, la bronchite chronique, une obstruction des petites voies aériennes et l'emphysème,
- des maladies obstructives ou inflammatoires des voies aériennes de n'importe quel type, étiologie ou pathogenèse, en particulier une maladie obstructive ou inflammatoire des voies aériennes qui est un membre choisi dans le groupe consistant en la pneumonie éosinophile chronique, une maladie pulmonaire obstructive chronique (COPD), la COPD qui comprend la bronchite chronique, l'emphysème pulmonaire ou la dyspnée associée ou non associée à la COPD, la COPD qui est **caractérisée par** une obstruction progressive irréversible des voies aériennes, le syndrome de détresse respiratoire de l'adulte (ARDS), l'exacerbation de l'hyperréactivité des voies aériennes suite à une autre pharmacothérapie et une maladie des voies aériennes qui est associée à l'hypertension pulmonaire,
- la bronchite de n'importe quel type, étiologie ou pathogenèse, en particulier la bronchite qui est un membre choisi dans le groupe consistant en la bronchite aiguë, la bronchite laryngotrachéale aiguë, la bronchite arachidique, la bronchite catarrhale, la bronchite croupeuse, la bronchite sèche, la bronchite asthmatique infectieuse, la bronchite productive, la bronchite staphylococcique ou streptococcique et la bronchite vésiculaire,
- l'asthme de n'importe quel type, étiologie ou pathogenèse, en particulier l'asthme qui est un membre choisi dans le groupe consistant en l'asthme atopique, l'asthme non atopique, l'asthme allergique, l'asthme bronchique atopique à médiation par IgE, l'asthme bronchique, l'asthme essentiel, l'asthme vrai, l'asthme intrinsèque provoqué par des perturbations physiopathologiques, l'asthme extrinsèque provoqué par des facteurs ambiants, l'asthme essentiel ayant une cause inconnue ou inapparente, l'asthme non atopique, l'asthme bronchitique, l'asthme emphysémateux, l'asthme induit par l'effort, l'asthme induit par un allergène, l'asthme induit par l'air froid, l'asthme professionnel, l'asthme infectieux provoqué par une infection par des bactéries, des champignons, des protozoaires ou des virus, l'asthme non allergique, l'asthme naissant, le syndrome de respiration sifflante du nourrisson et la bronchiolite,
- une lésion pulmonaire aiguë,
- la bronchiectasie de n'importe quel type, étiologie ou pathogenèse, en particulier la bronchiectasie qui est un membre choisi dans le groupe consistant en la bronchiectasie cylindrique, la bronchiectasie sacculée, la bronchiectasie fusiforme, la bronchiectasie capillaire, la bronchiectasie kystique, la bronchiectasie sèche et la bronchiectasie folliculaire.

13. Association d'un composé suivant l'une quelconque des revendications 1 à 9 ou de ses sels ou produits de solvatation pharmaceutiquement acceptables, avec un ou plusieurs autres agents thérapeutiques choisis entre :
(a) des inhibiteurs de 5-lipoxygénase (5-LO) ou des antagonistes de la protéine activatrice de 5-lipoxygénase (FLAP),
(b) des antagonistes des leucotriènes (LTRA), comprenant des antagonistes de LTB₄, LTC₄, LTD₄ et LTE₄,
(c) des antagonistes du récepteur d'histamine, comprenant des antagonistes de H1 et H3,
(d) des agents sympathomimétiques vasoconstricteurs agonistes des adrénorécepteurs α1 et α2 destinés à être utilisés comme décongestionnants,
(e) des agonistes de β₂ à brève ou longue durée d'action,
(f) des inhibiteurs de PDE, par exemple des inhibiteurs de PDE3, PDE4 et PDE5,
(g) la théophylline,
(h) le cromoglycate de sodium,
(i) des inhibiteurs de COX, consistant en inhibiteurs non sélectifs et inhibiteurs sélectifs de COX-1 ou COX-2 (médicaments anti-inflammatoires non stéroïdiens "MAINS"),
(j) des glucocorticostéroïdes oraux et inhalés,
(k) des anticorps monoclonaux actifs contre des entités inflammatoires endogènes,
(l) des agents anti-facteur de nécrose de tumeur (anti-TNF-α),
(m) des inhibiteurs de molécules d'adhésion, comprenant des antagonistes de VLA-4,
(n) des antagonistes des récepteurs de kinines B₁ et B₂,
(o) des agents immunosuppresseurs,
(p) des inhibiteurs de métalloprotéases de matrice (MMP),
(q) des antagonistes des récepteurs de tachykinine NK₁, NK₂ et NK₃,
(r) des inhibiteurs d'élastase,
(s) des agonistes du récepteur d'adénosine A2a,
(t) des inhibiteurs d'urokinase,
(u) des composés qui agissent sur les récepteurs de dopamine, par exemple des agonistes de D2,
(v) des modulateurs de la voie de NFκB, par exemple des inhibiteurs de IKK,
(w) des modulateurs de voies de transmission de cytokines, tels que les inhibiteurs de la kinase MAP p38, de la kinase syk ou de la kinase JAK,
(x) des agents qui peuvent être classés dans les agents mucolytiques ou agents antitussifs,
(y) des antibiotiques,
(z) des antagonistes des prostaglandines, tels que des antagonistes de DP1, DP2 ou CRTH2,
(aa) des inhibiteurs de HDAC,
(bb) des inhibiteurs de kinase PI3, et
(cc) des antagonistes de CXCR2.

14. Intermédiaire de formule 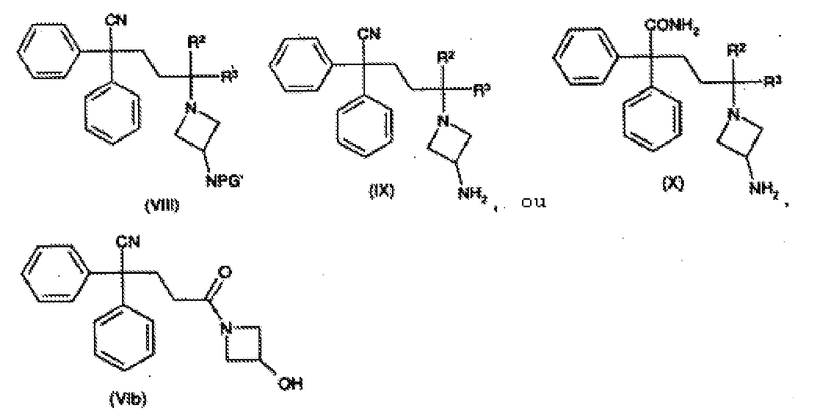 dans laquelle R² et R³ sont tels que définis pour les composés de formule (I) et PG' représente un groupe convenable protecteur de la fonction amine.
